# EUROPEAN PATENT SPECIFICATION

(11) **EP 2 646 050 B1**
(45) Date of publication and mention of the grant of the patent: **07.09.2016**
(21) Application number: 11802668.1
(22) Date of filing: 02.12.2011
(51) Int. Cl.: A61K 39/145, A61K 39/42, C07K 16/10, C07K 14/11

(54) **VACCINE AGAINST INFLUENZA H5N1 VIRUSES, MEDICAMENT AND TREATMENT OF H5N1 VIRAL INFECTIONS**
IMPFSTOFF GEGEN INFLUENZA VIREN H5N1, MEDIKAMENT UND BEHANDLUNG VON H5N1 VIRUSINFEKTIONEN
VACCIN CONTRE DES VIRUS H5N1 DE LA GRIPPE, MÉDICAMENT ET TRAITEMENT D'INFECTIONS VIRALES PAR H5N1

(30) Priority: 02.12.2010 DE 102010060988
(43) Date of publication of application: 09.10.2013
(73) Proprietor: mAB-Factory GmbH, 38108 Braunschweig (DE)
(72) Inventor: DÜBEL, Stefan, 38108 Braunschweig (DE); MERSMANN, Jana, 38108 Braunschweig (DE); MEIER, Doris, 37444 St. Andreasberg (DE)
(74) Representative: Margue, Robert Germain
(86) International application number: PCT/EP2011/071601
(87) International publication number: WO 2012/072788

(56) References cited:
- WO-A1-2009/119722
- WO-A2-2008/157419
- H.-L. J. OH ET AL: "An Antibody against a Novel and Conserved Epitope in the Hemagglutinin 1 Subunit Neutralizes Numerous H5N1 Influenza Viruses", JOURNAL OF VIROLOGY, vol. 84, no. 16, 2 June 2010 (2010-06-02), pages 8275-8286, XP55023354, ISSN: 0022-538X, DOI: 10.1128/JVI.02593-09
- H.-T. HO ET AL: "Rapid Detection of H5N1 Subtype Influenza Viruses by Antigen Capture Enzyme-Linked Immunosorbent Assay Using H5- and N1-Specific Monoclonal Antibodies", CLINICAL AND VACCINE IMMUNOLOGY, vol. 16, no. 5, 1 May 2009 (2009-05-01), pages 726-732, XP55023724, ISSN: 1556-6811, DOI: 10.1128/CVI.00465-08
- NIKOLAI V KAVERIN ET AL: "Epitope mapping of the Hemagglutinin molecule of a Highly Pathogenic H5NI Influenza Virus by Using Monoclonal Antibodies", JOURNAL OF VIROLOGY, THE AMERICAN SOCIETY FOR MICROBIOLOGY, US, vol. 81, no. 23, 1 January 2007 (2007-01-01), pages 12911-12917, XP008140175, ISSN: 0022-538X, DOI: 10.1128/JVI.01522-07
- KAVERIN: "Structure of antigenetic sites on the haeomagglutinin molecule of H5 avian influenza virus and phenotypic variation of escape mutants", JOURNAL OF GENERAL VIROLOGY, vol. 83, no. 10, 1 January 2002 (2002-01-01), pages 2497-2505, XP55023728, ISSN: 0022-1317
- IRINA A RUDNEVA ET AL: "Antigenic epitopes in the hemagglutinin of Qinghai-type influenza H5N1 virus.", VIRAL IMMUNOLOGY, vol. 23, no. 2, 1 April 2010 (2010-04-01), pages 181-187, XP55023732,
- PHILPOTT ET AL: "Hemagglutinin mutations related to attenuation and altered cell tropism of a virulent avian influenza A virus.", JOURNAL OF VIROLOGY, vol. 64, no. 6, 1 June 1990 (1990-06-01), pages 2941-2947, XP55023734, ISSN: 0022-538X
- JIAN LI ET AL: "Fine antigenic variation within H5N1 influenza virus hemagglutinin's antigenic sites defined by yeast cell surface display", EUROPEAN JOURNAL OF IMMUNOLOGY, vol. 39, no. 12, 1 December 2009 (2009-12-01), pages 3498-3510, XP55023737, ISSN: 0014-2980, DOI: 10.1002/eji.200939532
- YIXIN CHEN ET AL: "Broad Cross-Protection against H5N1 Avian Influenza Virus Infection by Means of Monoclonal Antibodies that Map to Conserved Viral Epitopes", THE JOURNAL OF INFECTIOUS DISEASES, vol. 199, no. 1, 1 January 2009 (2009-01-01), pages 49-58, XP55023361, ISSN: 0022-1899, DOI: 10.1086/594374
- YANG S G ET AL: "Construction and cellular immune response induction of HA-based alphavirus replicon vaccines against human-avian influenza (H5N1)", VACCINE, ELSEVIER LTD, GB, vol. 27, no. 52, 9 December 2009 (2009-12-09), pages 7451-7458, XP026789507, ISSN: 0264-410X, DOI: 10.1016/J.VACCINE.2009.05.014 [retrieved on 2009-05-29]
- HIOE ET AL: "Overlapping cytotoxic T-lymphocyte and B-cell antigenic sites on the influenza virus H5 hemagglutinin.", JOURNAL OF VIROLOGY, vol. 64, no. 12, 1 December 1990 (1990-12-01), pages 6246-6251, XP55023543, ISSN: 0022-538X
- KATZ ET AL: "Pathogenesis of and immunity to avian influenza A H5 viruses", BIOMEDICINE AND PHARMACOTHERAPY, ELSEVIER, FR, vol. 54, no. 4, 1 May 2000 (2000-05-01), pages 178-187, XP005888635, ISSN: 0753-3322, DOI: 10.1016/S0753-3322(00)89024-1

## Description

### FIELD OF INVENTION

The invention relates to the field of vaccines, antibodies and methods of diagnosing and treating influenza, particularly influenza caused by a member of the *Orthomyxoviridae.*

### BACKGROUND OF INVENTION

The highly variant influenza virus type A has a negative sense ribonucleic acid genome and because of the error-prone viral polymerase point mutations accumulate within the viral genome (antigenic drift). Rearrangements of entire gene segments further occur when two or more viruses have co-infected a host cell (antigenic shift) so that viruses with new antigenic patterns are formed (Webster R et al 1992. Evolution and ecology of Influenza A viruses. Microbiol. Rev. 56:152-79). Influenza A/H5N1 viruses caused pandemic outbreaks when the originally avian virus acquired the ability to cross species and infect humans. With its first occurrence in Hong Kong in 1997 the highly pathogenic avian influenza virus (HPAIV) of H5N1 subtype killed 318 out of 543 infected humans which represents a fatality rate of 59% (World Health Organization, Situation updates-avian influenza http://www.who.int/ csr/disease/avian_influenza /updates/en/ index.html, accessed April 8, 2011). This virus has also been devastating to the world's poultry industry (World Organisation for Animal Health, Office International des Epizooties, OIE, 2011a), Accelerated evolution of this influenza virus has been reported partly as result of transspecies transmissions, e.g. from wild birds to land-based domesticated poultry or from poultry to humans, or after infection of partially immunized birds. The circulation of this virus in different host species resulted in ten distinct phylogenetic clades (0 - 9) which are further diverted into subclades (World Health Organization (2011) *Antigenic and genetic characteristics of influenza A(H5N1) and influenza A(H9N2) viruses for the development of candidate vaccine viruses for pandemic preparedness.* *http:*//*www.who.int*/ *csr*/*disease*/ *avian influenza*/*guidelines* /*h5n1 virus*/ *en*/*index.html,* accessed March 8, 2011).

Immunization strategies against influenza are based on inactivated influenza virus preparations propagated in embryonated eggs or, more recently, in permanent cell cultures. This procedure is time and cost intensive and the used influenza strains need to be adjusted annually upon the respective circulating influenza strains. The protective immunity elicited by these vaccines is mainly based on neutralizing antibodies against hemagglutinin (HA), a homo-trimeric transmembrane glycoprotein present on the surface of the virus. 16 distinct serotypes of hemagglutinin are known. Each monomer of this glycoprotein consists of two sub-domains, a globular head domain (HA1) and a stem loop domain (HA2) (Isin B et al 2002. Functional motions of influenza virus hemagglutinin: a structure-based analytical approach. Biophys. J. 82:569-81). The globular head region contains the receptor binding domain (RBD), formed mainly by beta-sheets, and carries five immunogenic epitopes A-E. The stem loop domain is responsible for virus fusion with the cell membrane and anchors the HA complex in the viral envelope by a transmembrane domain. The stem loop domain contains two helices with phenylalanines (F) at positions 63 and 88. These phenylalanine rings are directed inwardly in the avian H5N1 and outwardly in the human H1 strains. Due to its presence on the viral envelope HA is therefore a primary target for the generation of antibodies for pathotyping and virus neutralization and for the design of antiviral vaccines and drugs.

Oh et al., 2010 (Journal of Virology, 84(16): 8275-8286) discloses antibodies directed to a conserved antigenic determinant, or epitope, located at residues 260-263 in the head domain of a H5 hemagglutinin molecule.

WO 2011/087839 A1 (Falkner et al) discloses a vaccinia virus vaccine based on a vector containing the genes of hemagglutinin and neuraminidase from influenza A virus. The vaccine elicits neutralizing antibodies against the challenge virus and against various epitopes of HA conserved across the H5 clades and subclades (Hessel A et al (2011) PLoS ONE 6(1): e16247. dol:10.1371/joumal. pone.0016247). Further vaccines with activity against influenza A class H5 viruses are described in WO2011/107961 A2 (Kwon HJ et al), EP1925318 A1 (Paul-Ehrlich-Institute), WO 2011/08735 A1 (Lam D et al), WO 2011/41691 A1 (Bishop G et al), WO2011/91255A1 (Berghman L et al), WO 2011/112671 A1 (Clegg C et al), WO 2011/136738 A1 (Mookkan P et al). Live vaccines however are inherently dangerous due to the risk of unexpected viral mutations. Current influenza vaccines however produce immunity against seasonal strains of influenza only. According to long-term studies vaccination against influenza must be done using mixtures of at least three different virus strains. Because of the high variability of influenza A viruses, the protective activity of these antibodies and vaccines is limited. No vaccines are at hand which could be used in case of a new highly pathogenic influenza virus of subtype H5Nx and there is little time available after the first occurrence of a new influenza virus and a pandemia. Also no methods are available which allow passive or active immunization against individual influenza virus subtypes. The prior art therefore represents a problem.

### SUMMARY OF INVENTION

The present disclosure provides an antigenic determinant (H5 epitope) of influenza hemagglutinin which is conserved over distant H5N1 lineages but not found in related hemagglutinin H1, H2 or H6. The specification further provides antibodies, antibody fragments and receptors against a conserved antigenic determinant of HA which have influenza A neutralizing activity.

A further aspect of the disclosure is a H5N1 specific vaccine on basis of said antigenic determinant. A use in passive vaccination is provided, i.e. by administering an effective amount of antibodies against said antigenic determinant.

Since H5N1 viruses have split into numerous lineages or clades, the disclosure further provides a new method of in vitro diagnosis of viruses of the H5N1 lineages by a use of the disclosed receptors or antibodies or antibody fragments against said conserved antigenic determinant.

A further aspect of the present disclosure relates to an antibody or antibody fragment which corresponding antigenic determinant comprises the following amino acid sequence
SEQ ID NO: 1 **FRNVVWLIKKNNTYPTIKRSY** or an essential fragment thereof. A preferred aspect of that disclosure is an antibody which respective antigenic determinant is substantially similar to a spatial structure located in the head domain of the H5 hemagglutinin. One of more amino acids may be replaced in that spatial structure without effecting the binding of the disclosed antibodies or the immunogenic effect.

A preferred aspect of the disclosure is an antibody or fragment thereof which antigenic determinant is a spatial structure based on the essential amino acid sequence
SEQ ID NO: 2 **FRNVVW**

The spatial structure can further be characterized by studies such as alanine walk, while the 5 amino acids *RNVVW* (SEQ ID NO:3) within SEQ ID NO: 2 represent the essential primary core of that conserved HA spatial structure.
SEQ ID NO: 3 **RNVVW**

Another aspect relates to an antibody or receptor which antigenic determinant is a spatial structure on a H5N1-subtype of hemagglutinin. Moreover, the disclosure provides antibodies and antibody fragments wherein the variable region of the Heavy Chain and the variable region of the Light Chain are contained in amino acid sequence of SEQ ID NO: 4
SEQ ID NO: 4

The corresponding nucleotide sequence covering the epitop-binding region reads as follows:
SEO ID NO: 5

Moreover, antibody clones are provided such as the most preferred JM08 (JM6_SC-D3) described below which form an immunological basis for HPAIV diagnostics and therapeutics. The present disclosure underlines that immune libraries from animals challenged with phylogenetically distant virus variants followed by phage display methods using distant virus antigens in separate pannings represent a promising strategy for identifying conserved functional epitopes of highly variant viral antigens.

Another aspect of the disclosure is a protein or peptide comprising one or more antigenic determinants comprising a
sequence as described in SEQ ID NO: 1 or SEQ ID NO:2 or SEQ ID NO:3.

The term antibody encompasses, if not indicated otherwise, regularly recombinant antibodies, antibody fragments, single chain antibodies and antibody fusions as well as receptors. Those molecules which can further be adapted by derivatization or modification or humanization. The person skilled in the art will appreciate that these binding molecules may be coupled with effective or non-active fragments of other proteins and molecules. The person skilled in the art will further appreciate the advantages by having the disclosed antibodies and binding molecules coupled to effector molecules.

The present disclosure further relates to the use of these antibodies, receptors, single-chain antibody fragments, antibody fragments and antibody fusions in medical diagnostics and most importantly for treating persons and animals afflicted of an influenza A/H5 infection comprising the administering of an effective amount of recombinant antibody molecules as described to obtain passive vaccination. The recombinant antibody molecule preferably is a humanized antibody or a fragment thereof. In this connection the present specification provides nucleic acid and amino acid sequences of antibodies as well as sequence data of their respective epitopes which can be used for construction and production of such antibodies.
SEQ ID NO:6 and SEQ ID NO:7 disclose the scFv-coding DNA and amino acid sequences of scFv clone no. 1 (JM7_B-G7). The respective the antigenic determinant VPKIATRSK (SEQ ID NO:8) was determined by epitope mapping with 15mer peptides 73-77 human H5 monomer having the amino acid sequences given in SEQ ID NO:9; SEQ ID NO:10; SEQ ID NO:11, SEQ ID NO:12 and SEQ ID NO:13. The scFv did not bind to peptides with the amino acid sequences of SEQ ID NO:9 and SEQ ID NO:13 but to peptides 74 to 76 (SEQ ID NO: 10, 11 and 12).
SEQ ID NO: 14 and SEQ ID NO:15 disclose the DNA and amino acid sequences of scFv clone 2 (JM7_S-F8).
SEQ ID NO:16 and SEQ ID NO:17 disclose the DNA and amino acid sequences of scFv clone 3 (JM7_H-F1) which is identical with clone 9 (JM7-03 ; JM7_BD5).
SEQ ID NO:18 and SEQ ID NO:19 disclose DNA and amino acid sequences of scFv clone 4 (JM7_S-A9).
SEQ ID NO:20 and SEQ ID NO:21 disclose DNA and amino acid sequences of scFv clone 10 (JM7_S-A10).
SEQ ID NO: 22 and SEQ ID NO:23 disclose DNA and amino acid sequences of scFv clone 6 (JM7_B-H2). The antigenic determinant of scFv clone 6 comprises the amino acid sequence of SEQ ID NO:8 as determined by epitope mapping. The scFv clone 6 bound to peptides 74 (SEQ ID NO:10), 75 (SEQ ID NO:11) and 76 (SEQ ID NO:12) of the H5 monomer.
SEQ ID NO: 24 and SEQ ID NO: 25 disclose DNA and amino acid sequences of scFv clone 7 (JM6_SC-H1).
SEQ ID NO: 26 and SEQ ID NO: 27 disclose DNA and amino acid sequence of scFv clone 8 (JM6_SC_D3). Its antigenic determinant on H5 monomer (A(Anhui/1/2005) as determined by epitope mapping is RNVVW (SEQ ID NO:2). The antibody fragment did not bind to peptides 55 (SEQ ID NO:32), 56 (SEQ ID NO:33) and 57 (SEQ ID NO:34) of H5, while binding to peptides 51 (SEQ ID NO: 28), 52 (SEQ ID NO: 29), 53 (SEQ ID NO:30) and 54 (SEQ ID NO:31). The minimal binding epitope is binding RNVWV (SEQ ID NO:3) as this scFv clone 8 also bound to H5N1 sequences where the (F) in the antigenic determinant was replaced by other amino acids.

Further useful DNA and amino acid sequences for a variable region of an antibody clone binding to an antigenic determinant of the HA of H5 virus are given SEQ ID NOs: 35 to 41. Their antigenic determinants or epitopes may further be used as an effective ingredient or as part of an active ingredient in a method of passive vaccination and in the in vitro diagnostic of influenza viruses.

Wherever an embodiment, example or preference is described or expressed herein in relation to one aspect of the present invention, it shall be understood that the embodiment, example or preference applies equally to all other aspects of the invention unless this is not technically feasible.

### BRIEF DESCRIPTION OF FIGURES

The invention will be described in greater detail below, without limitation and purely by way of illustration, with reference to the accompanying drawings, in which:

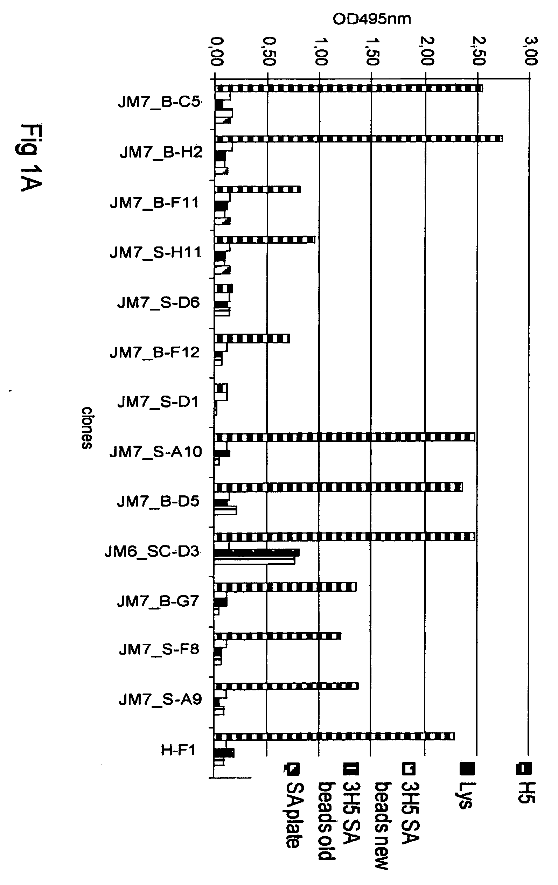

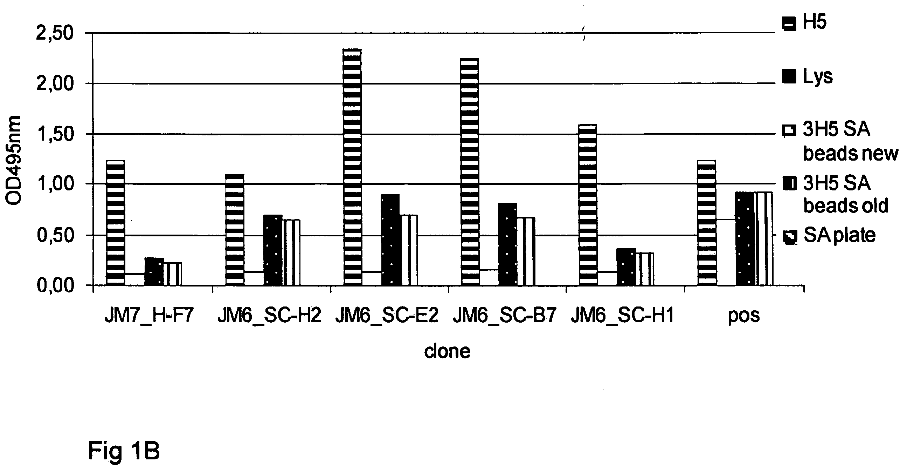

**Figures 1A and B** show block diagrams of an ELISA wherein scFv clones have been tested for their binding activity on H5 monomer of influenza AAnhui/1/2010 (dark blue) and trimeric H5 of influenza A/Canada goose/Germany/71/06 (yellow and lightblue) and lysozyme control (burgundy). 150ng per well H5 monomer of A/Anhui/1/2010 was immobilized on NUNC Maxisorp plates in 50mM carbonate buffer, pH 9.6 over night at 4°C. 20 µL trimeric H5 of NCanada goose /Germany/71/06, supernatant from a baculovirus expression system was immobilized on 1µL Streptavidin-coated beads per well. All values are means of duplicate measurements.

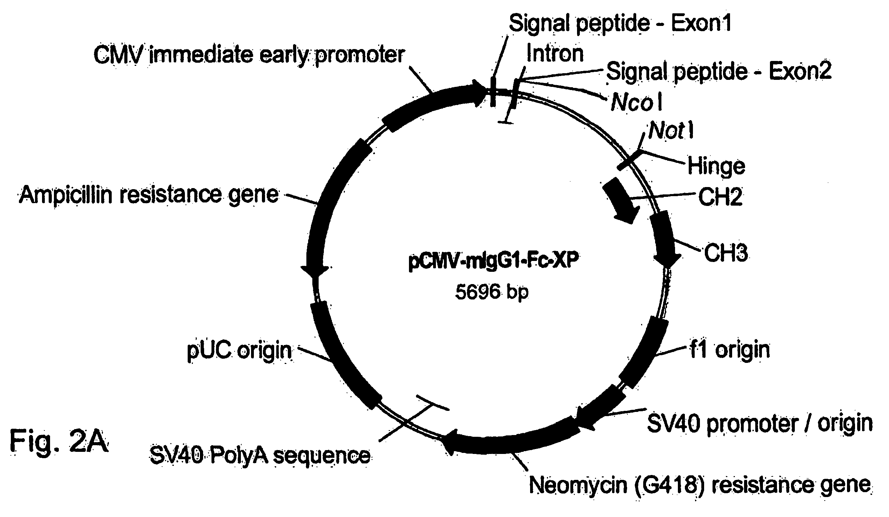

**Figure 2A** shows a diagram of expression vector **pCMV2.5 mlgG1 XP** used for expression of scFv-murine Fc-fusions in HEK 293-6E cells;

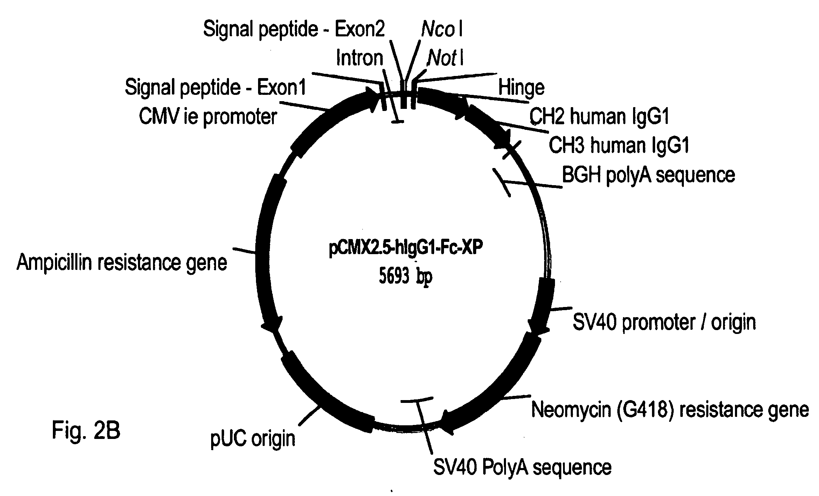

**Figure 2B** shows a diagram of expression vector **pCMX2.5 hlgG1 XP** used for expression of scFv-human Fc-fusions in HEK 293-6E cells.

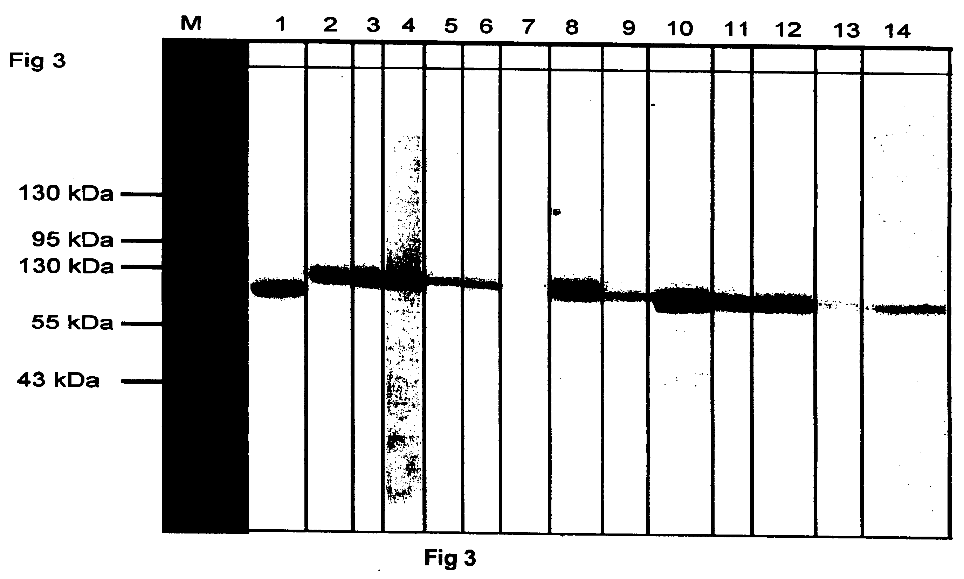

**Figure 3** shows an immunoblot analysis of scFv-human Fc-fusions on trimeric H5 (A/Canada goose/Germany/71/06 H5N1 HP); baculovirus expression supernatant was separated on a 10%polyacrylamide gel, blotted onto a PVDF membrane and bound scFv-humanFc-fusion antibodies were detected via chemiluminescence using HRP-labelled anti-human-Fc-antibody and ECL-detection kit; numbering of tested antibody clones is according to Table 5.

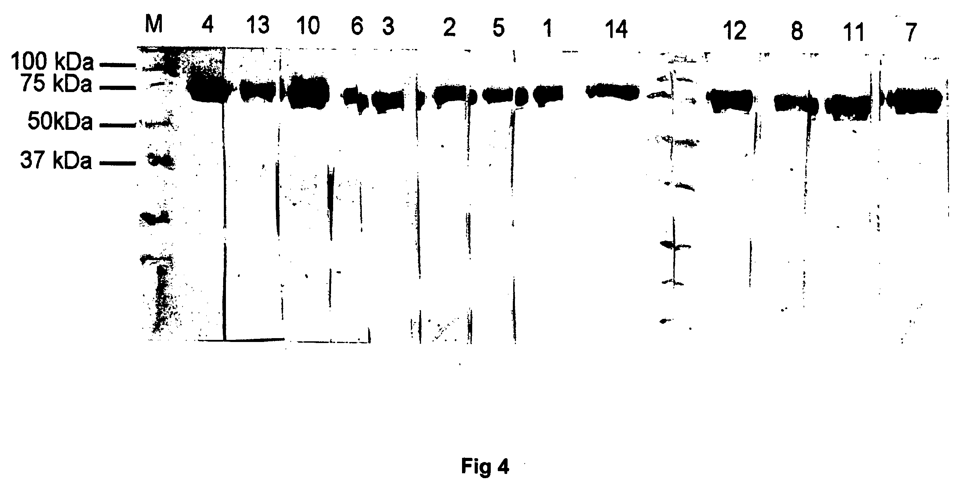

**Figure 4** shows an immunoblot analysis of scFv-human-Fc-fusion antibodies on monomeric human H5 (A/Anhui/1/2005, recombinant target; Sino Biologicals GmbH) as described with Figure 3.

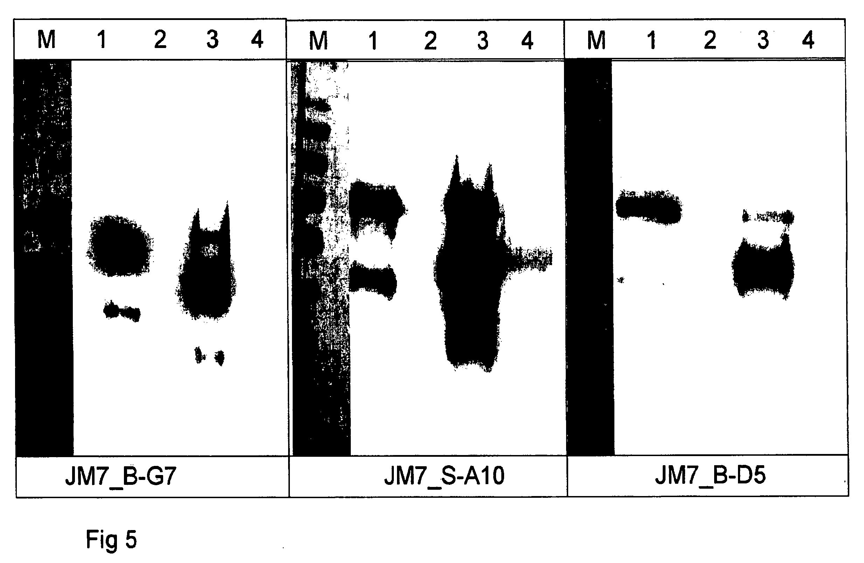

**Figure 5** shows an immunoblot analysis of individual scFv-human Fc-fusions on different targets. Different hemagglutinins of different preparations were separated on a 10% polyacrylamide gel and blotted onto a PVDF membrane M) protein ladder 1) trimeric avian H5 (A/Canada goose/Germany/71/06 H5N1 HP); 2) 3H7; 3) purified virus preparation A/Mallard/Ger/Wv478/04 (H5N2), 4) A/Vietnam/1197/2004.

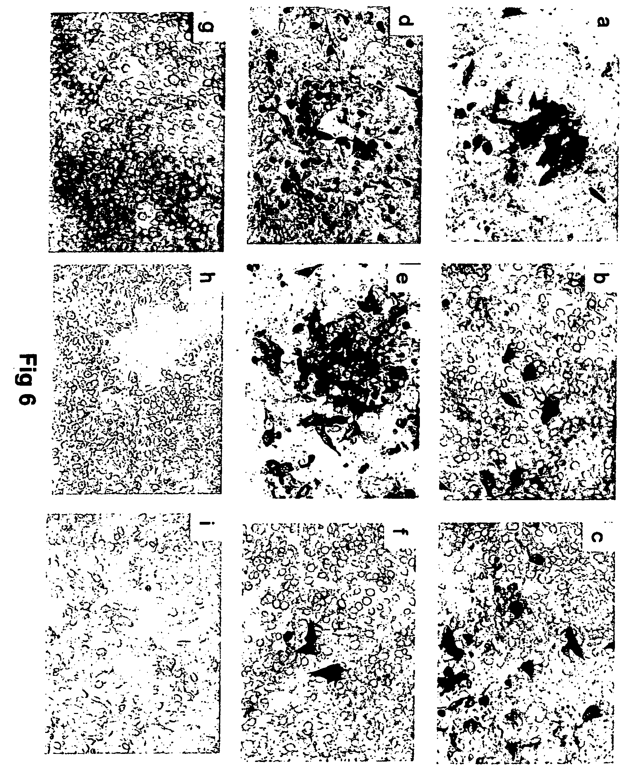

**Figure 6** shows the IPMA results for clone 8 (JM6_SC-D3)-part I. ScFv-human-Fc-fusion on STOMA24 (recombinant swine testis cells CCLV-RIE 1199 cells infected with a) A/teal/Föhr/Wv632/05 (H5N1, LP), b) A/chicken/Italy/22/98 (H5N9), c) A/duck/Potsdam/2216/84 (H5N6); d) A/turkey/Germany/617107 (H6N2) positive control anti-NP-antibody, e) A/mallard/B.C./544/05 (H5N9), f) A/goose/Manitoba/428/06 (H5N2), g) uninfected STOMA24 cells; h) Alturkey/Germany/617/07 (H6N2), i) A/chicken/Italy/22/98 (H5N9) negative control anti-human Fc-HRP-antlbody, .

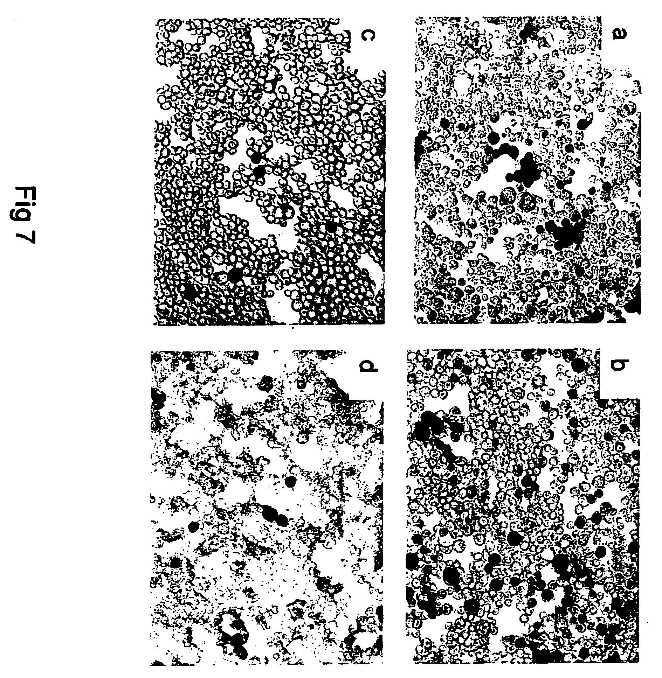

**Figure 7** shows the IPMA results for clone 8 (JM6_SC-D3)-partII. ScFv-human-Fc-fusion on High Five insect cells recombinant expressing H5 of a) A/chicken/Egypt/0879-NLQP/2008-R737/09 (H5N1 HP) b) A/Canada goose/Germany/71/06 H5N1 HP c) A/Canada goose/Germany/71/06 H5N1 HP detected by mAb 8292 (positive control) and d) A/chicken/Egypt/0879-NLQP/2008-R737/09 (H5N1 HP) detected by anti R737 chicken serum (positive control)

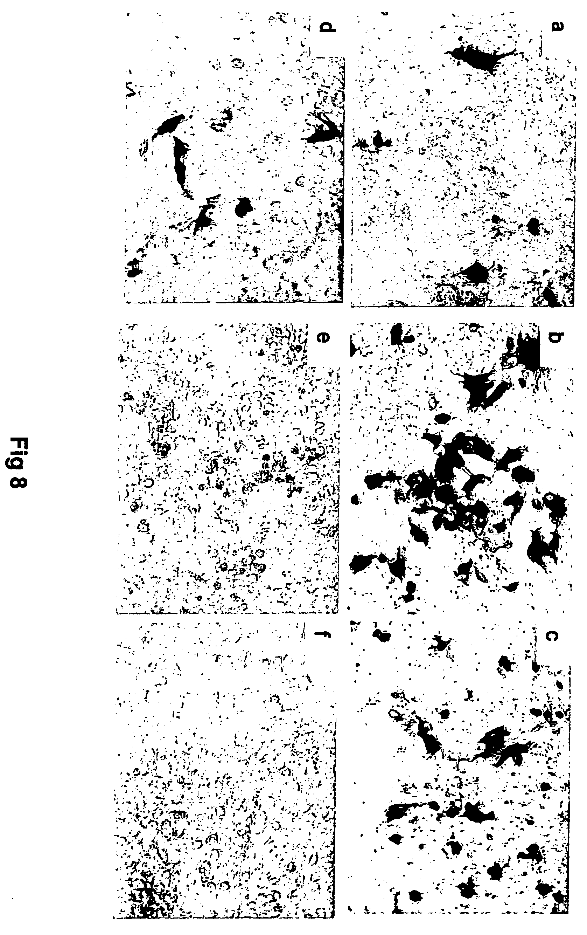

**Figure 8** shows the IPMA results for clone 1 (JM7_B-G7). ScFv-human-Fc-fusion on STOMA24 (recombinant swine testis cells CCLV-RIE 1199 cells infected with a) A/Mallard/Ger/Wv1349/03 (H5N3) b) A/teal/Föhr/Wv632/05 (H5N1, LP) c) A/duck/Potsdam/1402/86 (H5N2) d) A/goose/Manitoba/428/06 (H5N2) e) A/duck/Germersheim/R30/06 (H1N1) f)) uninfected STOMA24 cells; anti-human Fc-HRP background.

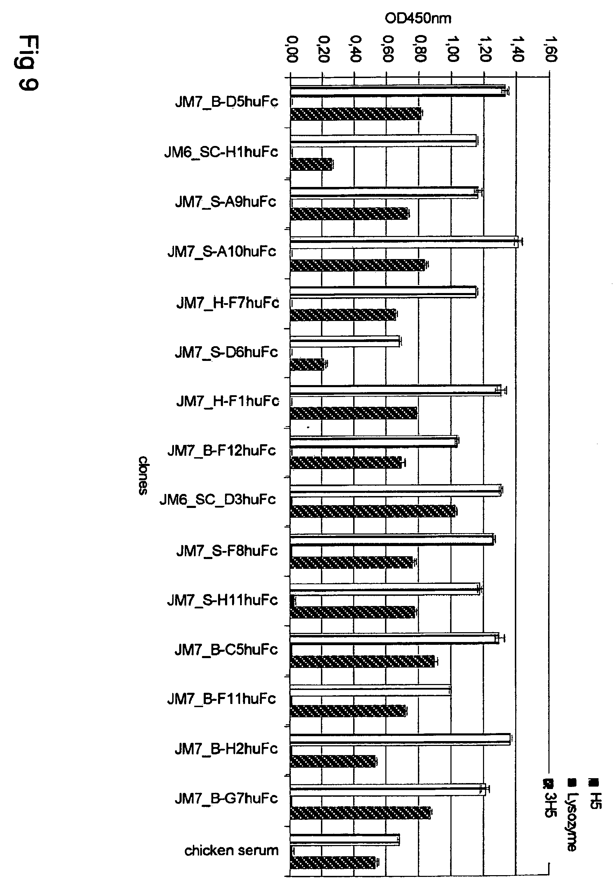

**Figure 9** shows the binding specificity of scFv-human- fusions after purification on monomer 125ng/ well H5 (A/Anhui/1/2005), on 125ng/well lysozyme (both immobilized on NUNC Maxisorp plates) and trimeric H5 (3H5; A/Canada goose/Germany/71/06) immobilized on streptavidin beads. 20µL baculovirus expression supernatant was incubated with 1µL SA beads for target immobilization. Bound scFv-murine-Fc fusions were detected via HRP conjugated anti-human-Fc-antibody. Staining was performed using TMB solution.

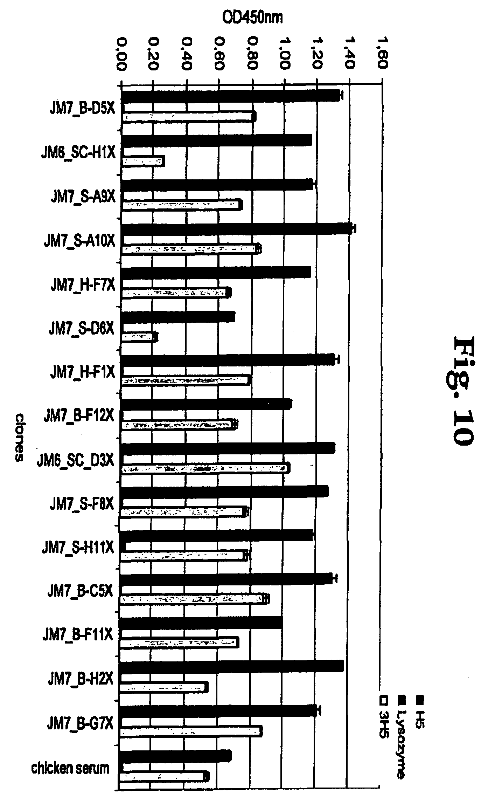

**Figure 10** shows the binding specificity of scFv-murine Fc- fusions after purification on monomer 125ng/ well H5 (A/Anhui/1/2005), on 125ng/well lysozyme (both immobilized on NUNC Maxisorp plates) and trimeric H5 (3H5; A/Canada goose/Germany/71/06) immobilized on streptavidin beads. 20µL baculovirus expression supernatant was incubated with 1µL SA beads for target immobilization. Bound scFv-murine-Fc fusions were detected via HRP conjugated anti-human-Fc-antibody.

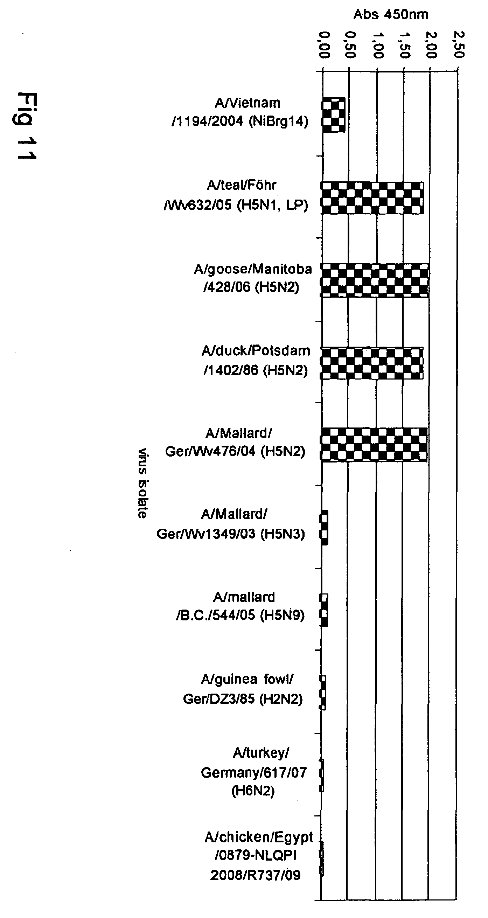

**Figure 11** shows a capture ELISA JM7_B-D5 humanFc/JM7_B-G7 murineFc with different virus preparations. 250ng per well of JM7_B-D5 human Fc fusions was immobilized to capture virus particles from H5 virus preparations produced in infected embryonated chicken eggs. Detection was performed using 250ng per well of clone JM7_B-G7 (murine-Fc-fusion), HRP-conjugated anti-murineFc-antibody and TMB solution. Values are means of duplicates.

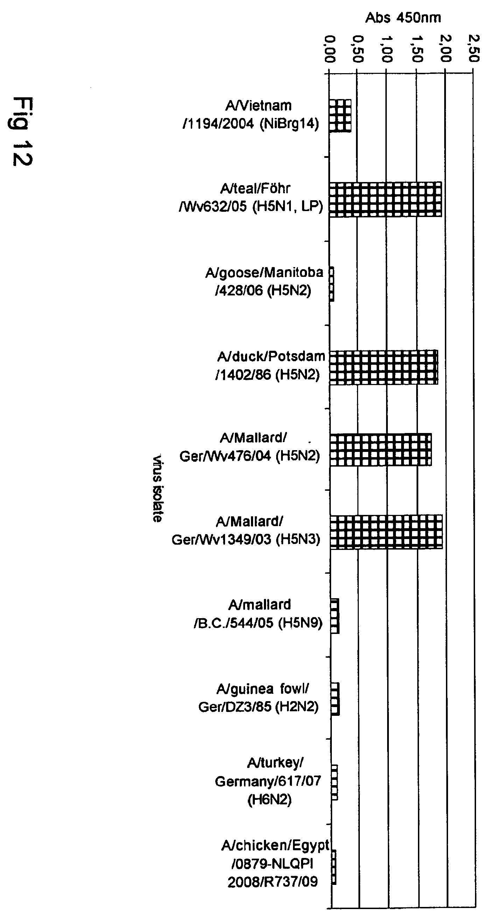

**Figure 12** shows a capture ELISA JM7_B-G7 humanFc/JM7_B-D5 murineFc as described above with Fig. 10, different virus preparations.

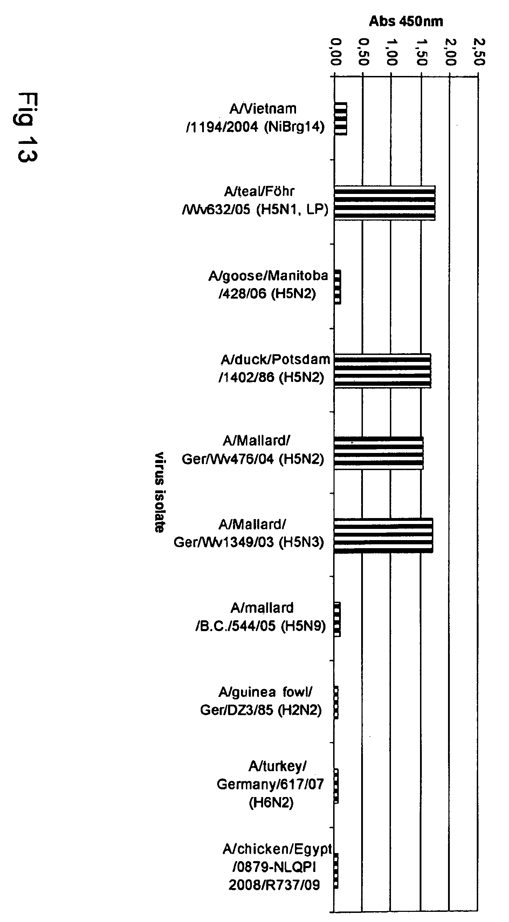

Figure 13 shows a capture ELISA JM7_B-G7 humanFc/JM7_H-F1 murineFc, with different virus preparations as described for Fig. 10.

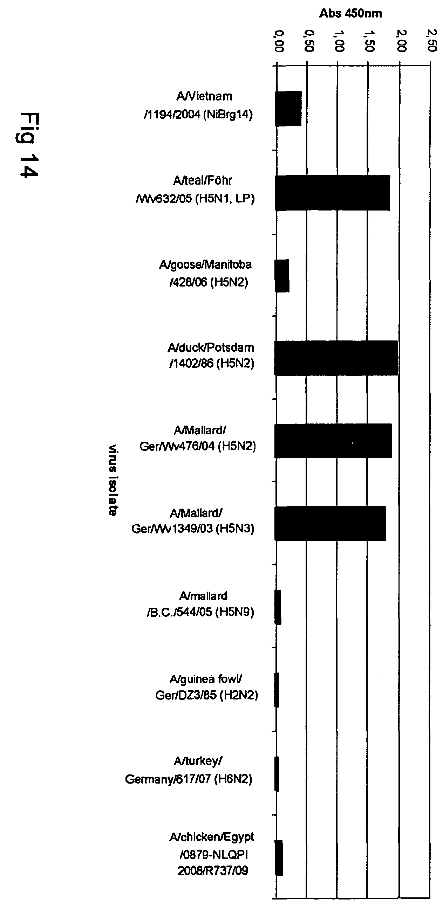

**Figure 14** shows a capture ELISA JM7_H-F1 humanFc/JM7_B-G7 murineFc with different virus preparations as described for Fig. 10.

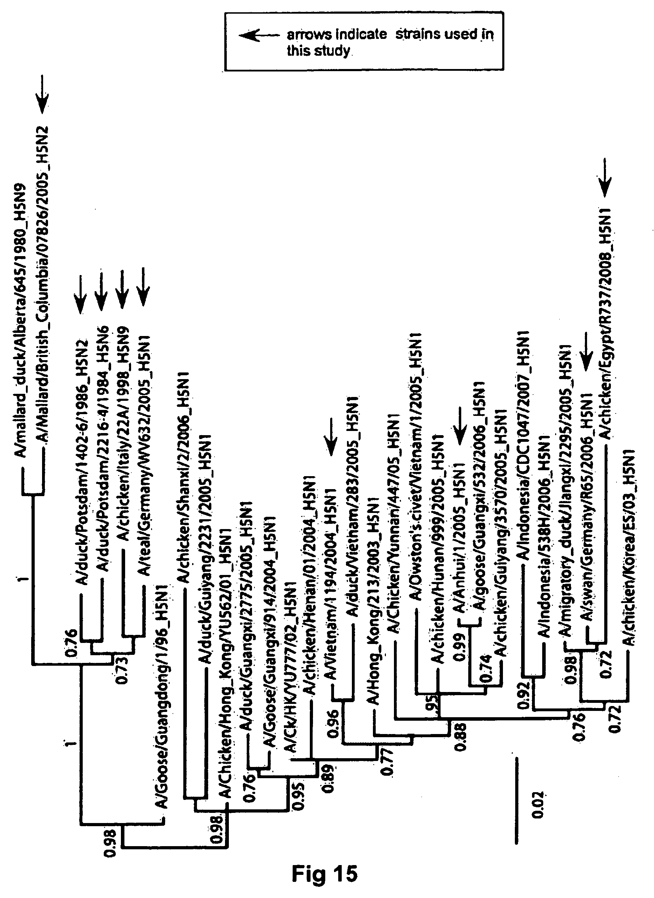

**Figure 15** shows the phylogenetic tree of influenza A virus subtype hemagglutinin protein sequences. The phylogenetic tree was constructed in a maximum likelihood framework (PhyML). Branch lengths are drawn in proportion to the scale bar. Isolates highlighted by red color have been analyzed in the current study. Blue numbers denote clades within the HPAIV H5N1 cluster as based on analyses of nucleotide sequences (WHO/FAO working group, ref.). Numbers at nodes represent the approximate probability of the robustness of the resulting branches (aRLT criterion).

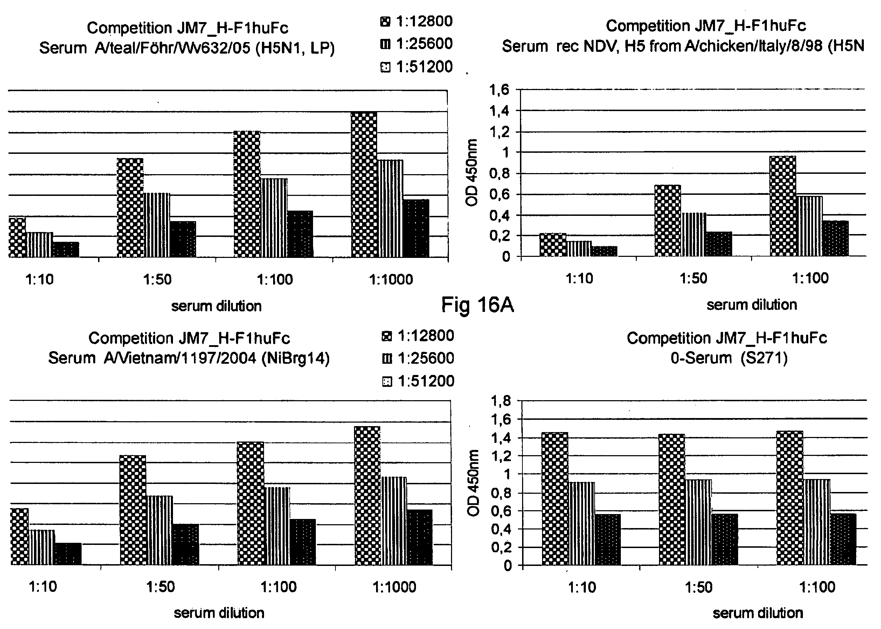

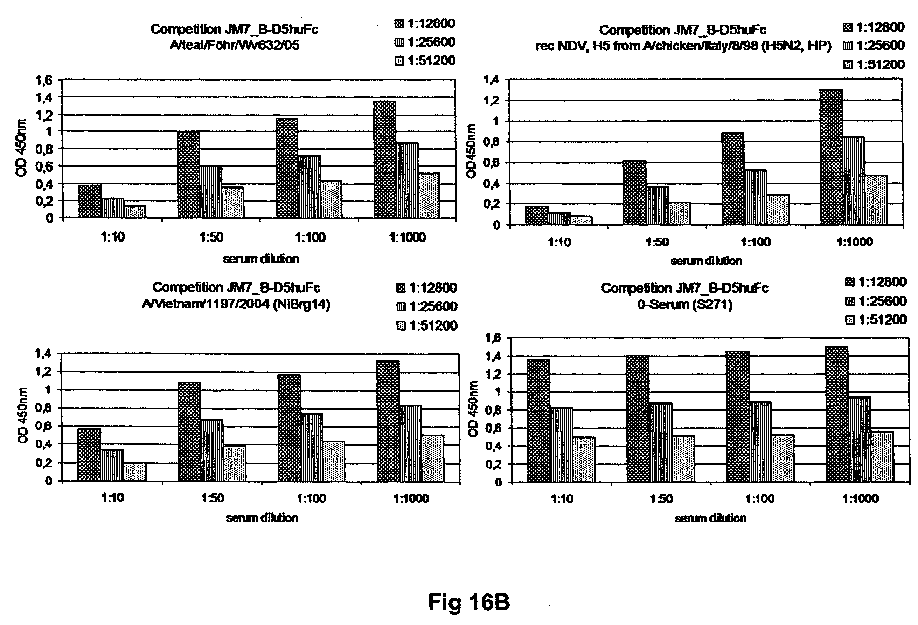

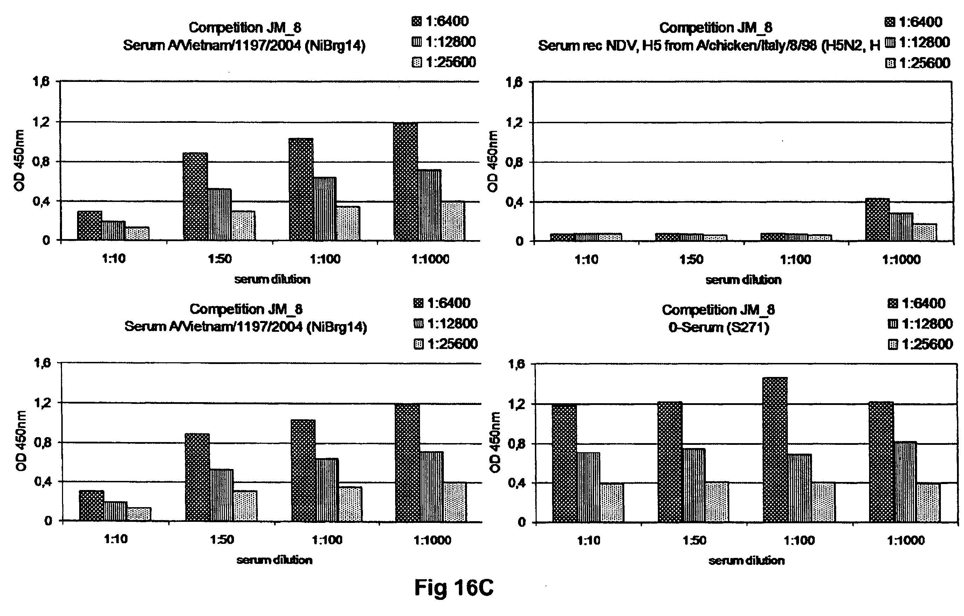

**Figure 16A-C** show a serum competition assay with antibody clone JM7_H-F1 human-Fc fusion, antibody clone JM7_B-D5 human-Fc-fusion, and antibody clone JM6_SC-D3 human-Fc-fusion, respectively;

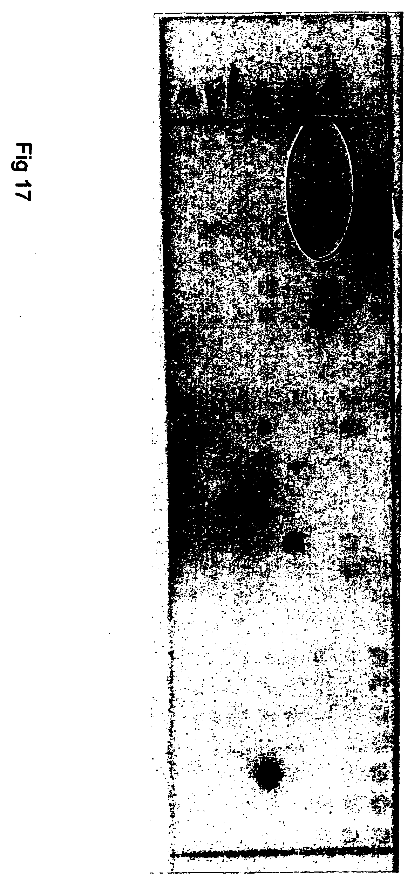

**Figure 17** shows the epitope mapping of clone JM08. 25 peptides are spotted per lane. Specific staining is detected for peptides No 51-54. The epitope of the scFv binder can therefore be determined as **(F)FRNVVW** (SEQ ID NO:2) according to the overlapping sequence of stained peptides.

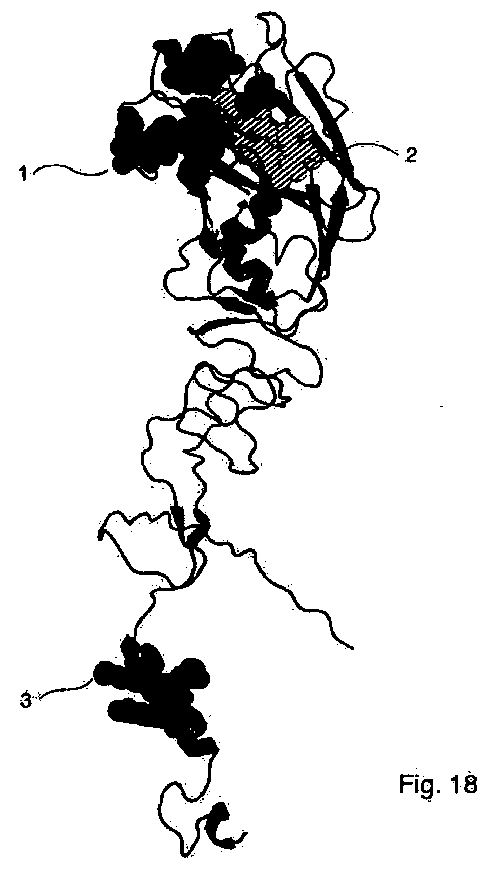

**Figure 18** shows the binding site of clone JM08. In red spheres antibody binding site, in blue spheres receptor binding sites according to Duvvuri et al 2009, cleavage site is indicated in cyan. Model constructed using POLYVIEW

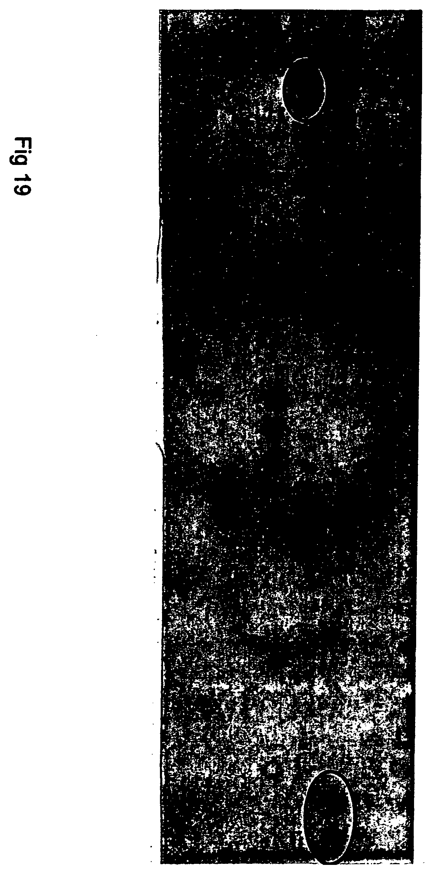

**Figure 19** shows the result of the epitope mapping of clone 6. Specific staining is visible for peptides No 74-76. The binding epitope can therefore be determined as **VPKIATRSK** (SEQ ID NO:8).

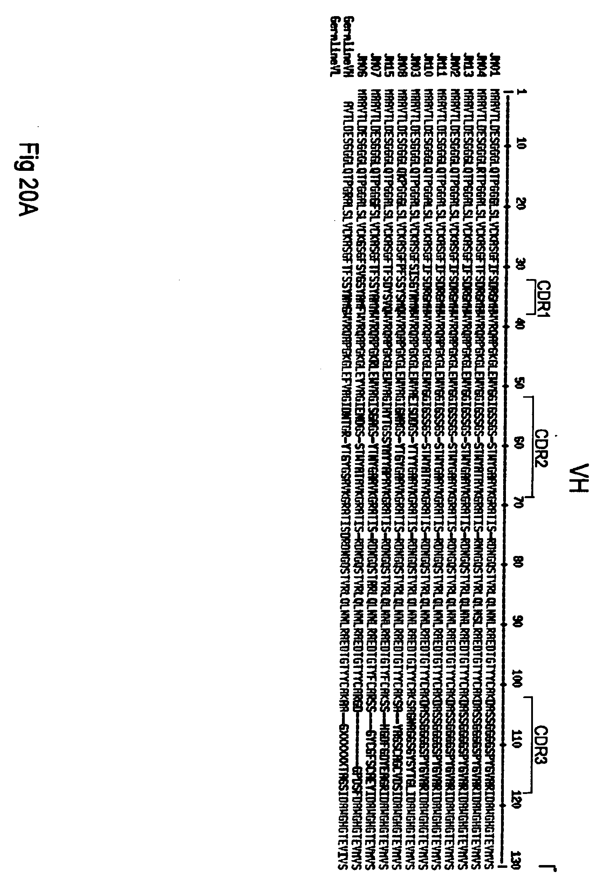

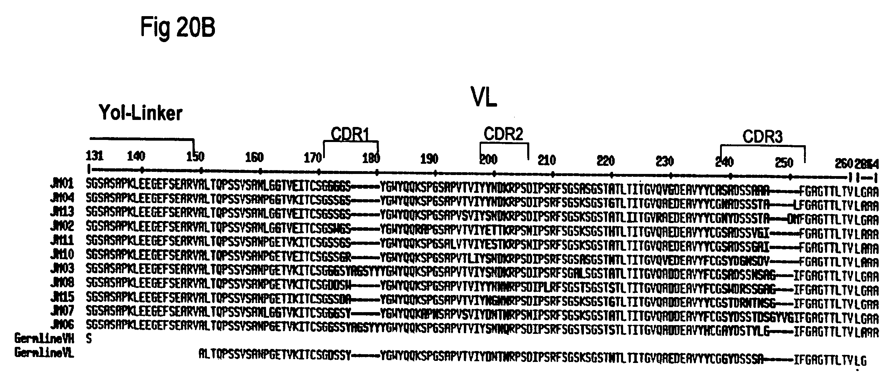

**Figure 20** shows an alignment of amino acid sequences of identified H5 binders compared to germ-line sequences of chicken antibodies. Amino acid identity to the germ-line sequences are shown in grey (cyan), differences in black, absence of corresponding residue by dashes. CDR and Yol-linker of scFv annotated. Germline VL is according to NCBI database AAA48861, germline VH according to Yamanaka, 1996.

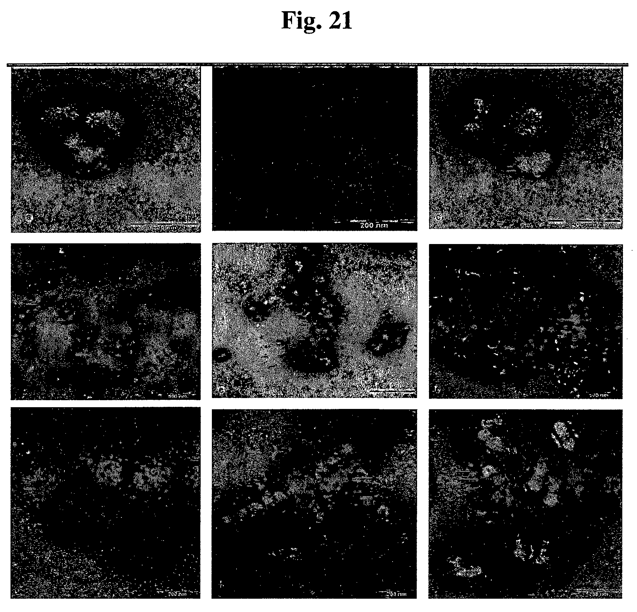

**Figure 21** shows electron micrographs of purified viruses (a-c), negative control with 15 nm protein A gold particles in MPBS (d) and antibody clones JM05 (e), JM08 (f), JM03 (g,h,K) with increasing amounts of antibody.

### DETAILED DESCRIPTION OF THE INVENTION

The Highly Pathogenic Avian Influenza Virus (HPAIV) H5N1 is very devastating to the world's poultry industry and poses a pandemic threat to humans. We have therefore screened the hemagglutinIn proteins (HA) of phylogenetically distant H5N1 viruses for antigenic determinants which are specific for H5N1 virus lineages and bound by recombinant antibodies with broad virus neutralizing effect.

The present specification discloses the construction of immune libraries from the antibody gene repertoire of chickens immunized with a mixture of influenza A/duck/Anhui/2006 (clade 2.3) and A/chicken/Shanxi/2/2006 (H5N1) and boosted with the Egyptian escape variant of influenza H5N1 (HPAIV A/chicken/Egypt/0879 - NLQP/2008 H5N1 clade 2.2.1) to obtain a repertoire library and recombinant antibodies with broad specificity to HA of very different H5N1 influenza strains. Other than mice chickens have the advantage of being the naive host of H5N1 viruses. Only two primer pairs are required to amplify the entire chicken antibody gene repertoire which simplifies the generation of large diverse immunoglobulin repertoires for use in antibody phage display methods. The specification describes screening procedures which force a selection of broadly specific recombinant antibodies. This is achieved by using HA proteins of different H5 variants in the pannings. This procedure resulted in eleven unique antibody clones which show specificity for different H5N1 clades with low or no cross-reactivity to HA of H1, H7 and H6 strains. Moreover, the application describes antibody clones which specifically detect H5N1 in different virus preparations, in infected cell culture and in electron microscopy. The antigenic determinants of the isolated antibody clones were further mapped by epitope mapping using peptide epitopes of HA H5 monomer. The specification discloses the amino acid sequence of an immunogenic linear epitope which is conserved in highly variant H5N1 clades including escape variants.

The obtained antibody clones were screened using two different recombinant H5 antigens and antibody clones selected as to whether they are specific to different H5N1 strains. We describe herein the isolation of an antibody clone named JM08 which binds to HA of all nine H5N1 clades (0, 1, 2.1, 2.2 and 2.2.1) but does not cross-react with H1-, H2- and H6-influenza viruses. Epitope-mapping revealed that the antigenic determinant of clone JM08 (JM6_SC_D3) is a linear contiguous epitope of five or six amino acids only in the globular head domain of the HA1 of H5 subunit. This epitope is highly conserved among H5N1 viruses including recent immune-escape mutants isolated from infected chickens in Egypt and because of its size resistant to mutational variations among all H5N1 virus strains. Moreover, we could show that antibodies against this conserved antigenic determinant have neutralizing effect. The present specification discloses the sequence of broadly specific antibody clones, and where possible their antigenic determinant on the H5 monomer. The combination of our screening methods resulted in an epitope present on HA of H5N1 clades which is linear and contiguous. This linear peptide epitope can be used in diagnostics and therapy of influenza as antibodies to this epitope were already found destroying and neutralizing H5N1 viruses. Antibodies against the antigenic determinant on HA can therefore be universally used in therapies against H5N1 infections on the basis of passive vaccination. The sequence of this linear epitope can further be used for the construction of an antigenic determinant for the development of immunogens and vaccines as well as in theraples for active vaccination.

The present disclosure further relates to vaccine strains comprising said neutralizing epitope which cover most, if not all variants within the H5N1 lineages. In order to demonstrate the broad protection *in vivo,* HA proteins of selected vaccine strains were expressed on the baculovirus surface and the efficacy of the vaccine formulations were then evaluated in a mouse model challenged with phylogenetically distant H5N1 strains. The described peptide epitope and the corresponding antigenic determinant on HA therefore represent targets for the development of avian influenza therapeutics and diagnostics.

### EXAMPLES

### MATERIALS AND METHODS

### Viruses and cells

The following viruses were provided by the repository of Friedrich-Löffler-Institute (FLI, Isles of Riems, Germany) and were all propagated in embryonated chicken eggs: A/Vietnam/1197/2004 (NiBrg14), A/chicken/Egypt/0879-NLQP/2008-R737/09 (H5N1 HP), A/Canada goose/Germany/71/06 (H5N1 HP), A/teal/Föhr/Wv632/05 (H5N1, LP), A/goose/Manitoba/428/06 (H5N2), A/duck/Potsdam/1402/86 (H5N2), A/Mallard/Ger/Wv476/04 (H5N2), A/chicken/Shanxi/2/2006 (H5N1), A/Mallard/Ger/Wv1349/03 (H5N3), A/duck/Pots-dam/2216/84 (H5N6), A/chicken/Italy/22/98 (H5N9), A/mallard/B.C./544/05 (H5N9), A/duck/Germersheim/R30/06 (H1N1), A/guinea fowl/Ger/D23/85 (H2N2), A/turkey/Germany/-617/07 (H6N2).

The human embryonic kidney (HEK) cell lines 293T (ATCC ® Number CRL-11268) and 293-6E (National Research Council (NRC), BRI, Montreal, Canada) were used for the recombinant production of single chain Fv-Fc antibodies. HEK293T was cultured in Dulbecco's modified Eagle's medium (DMEM, PAA, Pasching, Austria) supplemented with 8% (v/v) fetal calf serum (FCS, PAA) and 1% (v/v) penicillin/streptomycin (PAA) and cultured at 37°C and 7% CO₂. HEK293-6E cells were cultured in F17 (Freestyle version of CD17 medium, Invitrogen, USA) supplemented with 7.5 mM L-glutamine (PAA) and 0,1% (w/v) pluronic F68 in shake flask at 110 rounds per minute (rpm) with 5 cm orbital, 37°C and 5% CO₂ (Minitron, Infors, Bottmingen, Switzerland). The recombinant swine testis cell line Stoma 24 (CCLV-RIE 606) and High Five insect cells (CCLV-RIE 350) were obtained from the FLI (Riems, Germany).

### Virus preparation.

Viruses were propagated in 9 to 11 days old specific pathogen free embryonated chicken eggs and inactivated according to the OIE (2009). Allantoic fluid containing virus was harvested and centrifuged at 3500 round per minute (rpm) for 30 min. Clear supernatant was loaded onto a sucrose gradient (60%, 35% (w/v) with 5 mL of each sucrose fraction; sucrose dissolved in Tris-buffer (100 mM Tris pH7.4, 3 mM EDTA pH7.2) and centrifuged for 2 hours at 27,000 rpm and 4°C using a Beckmann LE-70 ultracentrifuge (rotor SW32) with Ultraclear centrifuge tubes (38.5 mL, SW28/32). Virus containing band was diluted 1:2 in Tris buffer and loaded onto a second sucrose gradient (60/50/40/30% (w/v) sucrose; 7 mL each fraction) and centrifuged over night at 27,000 rpm, 4°C. Virus containing bands were diluted 1:5 in 0.85% (w/v) NaCl and pelleted at 27,000 rpm, 4°C for 90 min. Virus pellet was finally resuspended in 0.85% NaCl.

### Recombinant H5 protein and anti-H5 sera

Recombinant monomeric H5 was obtained from Sino Biologicals Inc. (Avian Influenza Hemagglutinin H5N1, A/Anhui/1/2005, His tag, # 11048-V08H1; Lot MB03AP2801). Biotinylated recombinant trimeric H5 antigen of A/Canada goose/Germany/71/06 H5N1 was produced at the FLI using a baculoviral expression system. Anti-H5 chicken serum S89 was produced according to described standard procedures.

### Sequence analysis.

The amino acid sequences of the hemagglutinin (HA) protein of H5N1, H1N1, H2 and H6 viruses were retrieved from Influenza Virus Resource, a public database that gather influenza genome sequences from the National Institute of Allergy and Infectious diseases (NIAID) and the Genbank of the National Center for Biotechnology Information (NCBI). Sequences were aligned using MAFFT (Katoh et al., 2005) then viewed and edited using BioEdit version 7.0.5.3 (Hall, 1999).

### Structure modelling

The tertiary structure views of H5 glycoprotein monomer as well as epitopes mentioned here were generated with 3D-JIGSAW (Contreras-Moreira and Bates, 2002) and viewed and edited by Polyview 3D (Porollo et al., 2004). H5 numbering was based on the mature protein and functions of specific positions were defined after Duvvuri et al. (2009). Secondary structure was of the HA gene was predicted using Protein Structure Prediction Server "PSPIRED version 3.0" (Buchan et al., 2010).

### Modelling

Tertiary structure views of H5 glycoprotein monomer of selected sequence were generated with 3D-JIGSAW and viewed and edited by Polyview 3D. H5 numbering was based on the mature protein and functions of specific positions were defined after Duwuri et al. in Genomics Proteomics Bioinformatics (2009) 7(1-2):47-56.

### Phylogenetic analyses

In order to assess the diversity of the HA proteins of influenza A virus isolates within subtype H, we compiled an alignment (MUSCLE, Edgar, 2004) of 26 complete HA protein sequences representing relevant clades within subtype H5. Sequences from Europe, Asia and North America were retrieved from Genbank. Substitution models were fitted according to the Bayesian information criterion using ModelTest (Posada 2008) as implemented in Topali (Milne et al., 2009). Phylogenetic relationships based on the JTT+I+G model were estimated in a maximum likelihood (ML) framework using PhyML and the approximate Likelihood Ratio Test (aLRT) as well as by Bayesian inference (BI). BI was conducted using MrBayes (Huelsenbeck and Ronquist. 2001). The BI analysis chain was sampled every 10^{th} of a total of 10.000 generations with a burn-in of 250 trees.

### Transmission electron microscopy (TEM)

Thin carbon support films, approximately 10 nm thick, were prepared by sublimation of carbon from a carbon thread on to freshly cleaved mica (SCD500, Bal-Tec, Liechtenstein). Using 300 mesh nickel grids virus samples were negatively stained with 4% (w/v) aqueous uranyl acetate according to the method of Valentine (Valentine, R.C., Shapiro, B.M., Stadtman, E.R. 1968. Regulation of glutamine synthetase. XII. Electron microscopy of the enzyme of Escherichia coli., Biochemistry 7, 2143-2152). Antibody labeled virus samples were stained by placing a butvar-coated 300 mesh copper grid for 30 sec on a drop of the virus suspension, washed in TE buffer (10 mM trisHCl, 1 mM EDTA, pH8.0?) and then negatively stained with a 2% (w/v) aqueous uranyl acetate solution). Samples were examined in a Zeiss 910 TEM (Zeiss, Oberkochen, Germany) with an acceleration voltage of 80 kV at calibrated magnifications. Images were recorded digitally with a Slow-Scan CCD-Camera (ProScan, 1024x1024, Scheuring, Germany) with ITEM-Software (Olympus Soft Imaging Solutions, Münster, Germany).

For field emission scanning electron microscopy samples were adsorbed onto butvar-coated 300 mesh copper grids by a grid for 30 sec on a drop of the antibody labelled virus suspension, washed in TE-buffer (10 mM Tris.HCl, 1 mM EDTA, pH 7.6), washed in distilled water and subsequently air-dried. Grids were mounted on to placing conductive adhesive tabs on aluminium stubs. Samples were examined in a Zeiss DSM982 Gemini field emission scanning electron microscope (Zeiss, Oberkochen, Germany) using the Everhart-Thornley SE-detector and the inlens SE-detector in a 75:25 ratio at an acceleration voltage of 3 kV. Images were recorded on to a MO-disc.

### EXAMPLE 1 - Animal immunization and sample preparation.

Eight SPF chickens were immunized at the age of three weeks with a vaccine based on A/duck/Anhui/2006 (Chinese commercial inactivated vaccine Re-5), a highly pathogenic avian influenza virus (HPAIV) of subtype H5N1 which clusters into clade 2.3 and a co-vaccine based on A/chicken/Shanxi/2/2006 clustering into clade 7. Three weeks after the immunized birds were boosted with phylogenetically distant HPAIV A/chicken/Egypt/0879 - NLQP/2008 H5N1 virus. This virus of clade 2.2.1 represents a vaccine escape mutant recently emerging in vaccinated poultry in Egypt. All immunized birds survived without developing symptoms until day 14 after second immunization. The birds were then sacrificed and heparinised blood samples and spleens were obtained for isolation of RNA from lymphocytes and spleenocytes. All experimental procedures followed official German animal welfare regulations (permission LALLF 127M-V/TSD/7221.3-2.1-031/09).

### EXAMPLE 2 - Constriction of two separate anti-H5 chicken immune antibody libraries

The total RNA of peripheral blood lymphocytes (PBL) and spleen from immunized chickens was isolated using TRIZOL® (Sigma Aldrich) according to the manufacturer's protocol.

Library constructions were done to obtain separate anti-H5 chicken immune antibody-libraries from spleen (referred to as KFC_AIV2_S) and peripheral blood lymphocytes (referred to as KFC_AIV2_B). The RNA used was reverse transcribed using Superscript-II (Invitrogen, Darmstadt, Germany) and a random hexamer primer (denaturation 70°C for 5 min, synthesis 10 min 25°C, 50 min 42°C and finally 15 min 70°C). Chicken specific IgG-primers (Table 1) modified for our phage vector system (pIII) were used to amplify variable (V) heavy (H) and light (L) chains (Go-Taq-DNA polymerase, Promega; 25 cycles) in a first PCR.

**TABLE 1**

| *Primers for the construction of chicken immune library* | | |
|---|---|---|
| Primer name | Primed region | Primer Sequence 5' ---► 3' |
| MHChickVH_f: | Variable heavy chain forward | **GCC GTG ACG TTG GAC GAG TCC (SEQ ID NO:7)** |
| MHChickVH_r2: | Variable heavy chain reverse | **GGT TGC ACT CCC GGA GGA GAC CAT GAC TTC GGT CC (SEQ ID NO:8)** |
| MHChickVL_f: | Variable light chain forward | **GCG CTG ACT CAG CCG TCC TCG (SEQ ID NO:9)** |
| MHChickVL_r | Variable light chain reverse | **ACC TAG GAC GGT CAG GGT TGT C (SEQ ID NO:10)** |
| MHChickVH-NcoI_f: | Variable heavy chain forward | **GTCCTCGCA CC ATG GCC GCC GTG ACG TTG GAC GAG TCC (SEQ ID NO:11)** |
| MHChickVH-HindIII_r2 | Variable heavy chain reverse | **GTCCTCGCAAAG CTT TGG GGC GGA TGC ACT CCC GGA GGA GAC (SEQ ID NO:12)** |
| MHChickVL-MluI_f: | Variable light chain forward | **ACCGCCTCC A CGC GTA GCG CTG ACT CAG CCG TCC TCG (SEQ ID NO:13)** |
| MHChickVL-NotI_r: | Variable light chain reverse | **ACCGCCTCC GC GGC CGC ACC TAG GAC GGT CAG GGT TGT C (SEQ ID NO:15)** |

Restriction and cloning sites *(Nco*l*Hind*III for VH and *Mlu*I/*Not*I for VL) were inserted by a second PCR (Go-Taq-DNA polymerase, Promega; 25 cycles). Both V regions were cloned into vector pHAL14 (cf. Hust M & Dübel S, Phase display vectors for the in vitro generation of human antibody fragments in Methods Mol Biol 2005; 295:71-96; Hust M et al. J Biotechnol. (2011) 152(4):159-70) to obtain single chain Fv antibody genes genetically linked to the phage gIII gene encoding the major coat protein **pIII** to allow antibody display on the phage surface. The ligation products were transformed into *Escherichia coli* XL1-blue (Agilent Technologies, Böblingen, Germany), amplified and purified.

The resulting two scFv antibody gene libraries KFC-AIV2-spleen and KFC-AIV2-blood comprised 1.6x10⁷ and 5.7x10⁶ independent clones, respectively. Colony PCR of 50 clones per library revealed 100% and 97% full-size inserts corresponding to the expected size of antibody gene fragments for the KFC-AIV2-spleen and KFC-AIV2-blood library. Moreover, immunoblotting using an pIII specific mAb revealed high display of scFv fragments on phage for both libraries (packaged with helper phage M13K07 for monovalent display) represented by the protein band of scFv fused to the phage major coat protein pIII corresponding to an apparent molar mass of 100-110 kDa. These results underlined the high quality of both immune libraries,

### EXAMPLE 3 - Antibody phage display

### Strategy

Monomeric and biotinylated trimeric H5 proteins were used as antigen target in separate panning cycles. The rationale of this strategy was that an antibody would only successfully pass the selection when it reacts with both (all) panning antigens. The use of phage display instead of a screening of individual clones with different antigens allows for a dramatic reduction of screening work since the tedious analysis of individual clones is restricted to scFv fragments which react with both antigens. Instead of determining an epitope by panning on short peptides, we extended this approach to common antigenic determinants on HA by using five different and phylogenetically distant H5 variants, three of which were used in the immunization process, whereas the two others were used in the panning to increase the chances for finding broadly reactive antibodies. Finally for further analyses only those scFv binders were considered and characterized that showed binding to both panning targets. By this panning and screening strategy we selected for only those scFv binders which recognize a common antigenic determinant on phylogenetically distant H5 viruses.

As the immobilization strategy of the panning target influences the epitopes recognized by phage display derived scFv binders we further tried to preserve the conformation of the antigens as much as possible by immobilizing them on magnetic beads. This was supposed to support the identification of scFv binders binding to conserved conformational epitopes, in addition to linear epitopes, since we were uncertain whether the HA of distant H5N1 strains would bear a common antigenic determinant at all.

### Amplification of phages

Cells were scraped from the plates and 40mL dYT-medium containing 100µg/mL ampicillin and 100mM glucose were inoculated with an OD 600nm of 0.15 and grown at 37°C, 250 rpm. At OD 600nm= 0.5 5mL of the cultures were superinfected with 5x10¹⁰ pfu M13K07 helper phage and further incubated at 37°C, 110rpm for 30 minutes. Culture was centrifuged at 4000 rpm and room temperature for 10 minutes and the pellet resuspended in 40mL dYT-medium, 100µg/mL ampicillin. Phages were produced over night at 30°C and 250 rpm.

### Preparation of phages

Cells were harvested at 13,000 rpm, 4°C for 10 minutes. 30 mL of the supernatant were mixed up with 7.5mL 5x PEG/NaCl and incubated on ice for 1h. After centrifugation at 13,000 rpm, 4°C for 30 minutes pellet was resuspended in phage dilution buffer. Suspension was cleared via centrifugation at 13,000 rpm, 4°C for 10 minutes and phage preparation was stored at 4°C.

### Bead preparation

Recombinant monomeric H5 panning target (Sino Biologicals GmbH; Lot MB03AP2801; recombinant antigen H5 A/Anhui/1/2005) was immobilized to Dynabeads® M270 Carboxylic Acid (Invitrogen, Darmstadt, Germany) according to manufacturer's procedures. A total of 10µL beads was activated using N-hydroxysuccinimide/1-ethyl-3-[3-dimethylaminopropyl]carbodiimide hydrochloride (NHS/EDC) for 8 minutes at room temperature. After two short wash steps with 10mM sodium acetate buffer, pH 5.0 per 10µL magnetic beads 500ng of recombinant H5 were immobilized in 10mM sodium acetate buffer, pH 5.0 for 20 minutes in a final volume of 120 µL. Subsequently beads were washed with PBS and blocked with ethanolamine for 1 h at room temperature. Finally beads were washed with PBST (PBS containing 0.05% (v/v) Tween®-20, Serva, Heidelberg, Germany) and blocked with PBST supplemented with 1% (w/v) bovine serum albumin (BSA) for 1 h at RT.

Biotinylated recombinant trimeric H5 was immobilized to Dynabeads® M270 Streptavidin (Invitrogen, Darmstadt, Germany) according to manufacturer's coupling procedures. Briefly, 10 µL streptavidin beads were incubated with in 200 µL baculovirus-expression supernatant for 1 h at RT.

### First panning on monomeric H5

For the first panning round 5x10¹¹ cfu phages of each of the two chicken immune libraries KFC_AIV2_S and KFC_AIV2_B were incubated separately with prepared H5 beads for 80 minutes at room temperature in 1% BSA PBST 0.05%. Thereafter, the beads were washed 15 times with 800 µL PBST. Bound phage were eluted from the beads by incubation with 200 µL elution buffer, 10 µg/mL trypsin for 30 min at 37°C, 600 rpm. 5 mL *E. coli* XL1-blue-MRF' (Agilent Technologies, Böblingen, Germany), 2xYT medium supplemented with 20 µg / mL tetracycline at 250 rpm at 37°C until reaching the logarithmic growth phase (OD _{600 nm} = 0.5), were infected with the eluted phage by incubation for 30 min at 37°C and shaking at 110 rpm. Cells were plated onto 2xYT-agar plates supplemented with 100 mM glucose (G), 100 µg/mL ampicillin (A) and 20 µg/mL tetracycline (T) (2xYT-GAT). Plates were incubated over night at 37°C.

### Amplificafion of phages

Colonies were scraped off the plates with a spatula in 5 mL medium. A few hundred µL of this suspension was inoculated with 40 mL 2xYT-GA medium to obtain an initial OD _{600 nm} of 0.15. The cells were incubated at 37°C and 250 rpm until reaching an OD _{600 nm} of 0.5. Then, 10 mL of the cultures was superinfected with 5x10¹⁰ cfu M13K07 helper phage at 37°C for 30 min and 110 rpm. Culture was centrifuged at 4,000 rpm at room temperature for 5 min and the supernatant discarded. The pellet was resuspended in 40 mL 2xYT-medium containing 100 µg/mL ampicillin, 20 µg/mL tetracyclin and 100mM glucose (2xYT-GAT). Antibody phages were produced over night at 30°C and 250 rpm. Cells were removed by centrifugation at 13,000 rpm, 4°C for 10 min. A total of 30 mL of the phage containing supernatant was mixed with 7.5 mL 5x PEG/NaCl (20% (w/v) PEG 6000, 2.5M NaCl) and incubated on ice for 1 hour. After centrifugation at 13,000 rpm, 4°C for 30 min, the pellet was resuspended in phage dilution buffer (10 mM Tris-HCl, pH 7.5, 20 mM NaCl, 2 mM EDTA). Suspension was cleared via centrifugation at 13,000 rpm, 4°C for 10 min and phage preparation was stored at 4°C for the next panning round.

### Second panning round

For second panning round phages and beads were incubated in 1% milk PBST 0.05%. For KFC-AIV2-S 1x10¹¹ cfu phages were used, for KFC_AIV2-B 5x10¹⁰ cfu phages were used. Beads were washed 25 times using 800µL PBST 0.05% each time. Two successive rounds of pannings were performed using both anti-H5 chicken immune libraries KFC_AIV2-B and KFC_AIV2_S separately.

### Panning on biotinylated trimeric H5

Streptavidin beads (Invitrogen, Darmstadt, Germany) were coated with recombinant biotinylated trimeric H5 of A/Canada goose/Germany/71/06 H5N1 for 60 min at RT in PBST. This target was produced in a baculoviral expression system and delivered as culture supernatant after dialysis against PBS. Thereafter, the beads were washed with PBST and blocked in PBST supplemented with 2% milk powder (2%M-PBST) in the first panning round or in PBS with 2% BSA in the second round. The immune libraries (2) were diluted in blocking buffer and incubated with antigen loaded and blocked beads. Washing, elution and phage recovery procedures were performed according to procedures described in Panning 1. Two successive rounds of pannings were performed using both anti-H5 chicken immune libraries KFC_AIV2-B and KFC_AIV2_S.

### Screening

After pannings single clones were picked in 120µL dYT containing 100µg/mL ampicilline and 100mM glucose and cultured in the MTP 96 well format at 37°C and 850 rpm in MTP-shaker for 8 h. 20µL of each well were transferred to a new well containing 180µL dYT containing 100µg/mL ampicilline and 100mM glucose and incubated for 2h at 37°C and 850rpm in MTP-shaker. Cells were harvested by centrifugation at 3200xg, room temperature for 10 minutes. Supernatant was discharged and pellet was resuspended in 200µL per well dYT containing 100µg/mL ampicilline and 50µM IPTG to induce production of single chain antibody fragments. Production cultures were incubated at 30°C and 850 rpm in MTP-shaker over night. Next day cells were harvested by centrifugation at 3200xg, room temperature for 10 minutes and the single chain fragments in the supernatants tested by the combination of methods described below.

### Isolation of recombinant scFv against H5

We used two different H5 subtypes in separate panning campaigns, monomeric H5 immobilized to carboxylic magnetic beads and trimeric H5 immobilized to streptavidin magnetic beads. After each panning 96 antibody clones were isolated and produced as monoclonal soluble scFvs for antigen binding analysis using ELISA. Screening results are summarized in Tables 2 and 3.

**TABLE 2**

| *Panning results on monomeric H5* | | | | |
|---|---|---|---|---|
| Name of sublibrary | Analyzed scFvs Clones | Monoclonal binders on H5 monomer (plates) | Monoclonal binders on H5 monomer (Carboxy-beads) | Monoclonal binders on H5 monomer (on beads and plates) |
| KFC_AIV2_Blood | 96 | 82 | 67 | 67 |
| KFC_AIV2_Spleen | 96 | 81 | 72 | 71 |

**TABLE 3**

| *Panning results on trimeric H5* | | |
|---|---|---|
| Name of sublibrary | Analyzed scFvs Clones | Monoclonal binders to H5-trimer (streptavidin beads) |
| KFC_AIV2_Blood | 96 | 45 |
| KC_AIV2_Spleen | 96 | 58 |

From pannings against monomeric H5 a total of 82 and 81 clones of KFC-AIV2-spleen and KFC-AIV2-blood, respectively, bound to monomeric H5 antigen directly immobilized onto the ELISA plate. In case of monomeric H5 antigen covalently conjugated to magnetic beads, only 67 and 72 binders were identified. In total, 67 and 71 antibody clones recognized immobilized monomeric H5 antigen by both techniques. From pannings against trimeric H5, 45 and 58 antibody clones of KFC-AIV2-spleen and KFC-AIV2-blood, respectively, were found to bind biotinylated trimeric H5 antigen Immobilized on streptavidin.

### EXAMPLE 5 - DNA-fingerprinting of antibody clones

To identify unique antibody clones, after screening scFv genes were amplified by colony PCR with primers MH_LacZpro_f and MHgIII_r., which are specific for pHAL14 phage display. PCR fragments were digested with BstNI (NEB, Frankfurt/M., Germany) and analysed on 16% polyacrylamide gel (triborate-EDTA (TBE) buffer). Clones with different restriction patterns were subjected to DNA sequencing (GATC Biotech AG, Konstanz, Germany) to identify unique antibodies. We did not use a back-up Alu I digestion to investigate clones with identical BstN1 restriction patterns so that we cannot compare the two panning strategies and chicken antibody immune libraries as to antibody outputs.

After Bst NI digestion and DNA sequencing, 15 different antibody clones from both immune libraries showed specific binding to monomeric and trimeric H5. These antibody clones were isolated and subjected to further characterization. For reasons of confidentially we renamed isolated clones of scFv prior to sequencing as shown in the accordance table below:

**TABLE 4**

| *Accordance table of antibody names and numbers* | | | |
|---|---|---|---|
| Antibody number | Antibody name | Repertoire library | Panning target |
| 1 | JM7_B-G7 | KFC_AIV2-B | trimerized H5 |
| 2 | JM7_S-F8 | KFC_AIV-S | trimerized H5 |
| 3 | JM7_H-F1 | KFC_ AIV-S | trimerized H5 |
| 4 | JM7_S-A9 | KFC_AIV-S | trimerized H5 |
| 5 | JM7_S-H11 | KFC_AJV_S | trimerized H5 |
| 6 | JM7_B-H2 | KFC_AIV2-B | trimerized H5 |
| 7 | JM6_SC-H1 | KFC_AIV-S | H5 monomer |
| 8 | JM6_SC-D3 | KFC_AIV-S | H5 monomer |
| 9 | JM7_B-D5 | KFC_AIV2-B | trimerized H5 |
| 10 | JM7_S-A10 | KFC_AIV-S | trimerized H5 |
| 11 | JM7_H-F7 | KFC_AIV-S | trimerized H5 |
| 12 | JM7_B-C5 | KFC_AIV2-B | trimerized H5 |
| 13 | JM7_B-F11 | KFC_AIV2-B | trimerized H5 |
| 14 | JM7_B-F12 | KFC_AIV2-B | trimerized H5 |
| 15 | JM7_S-D6 | KFC_AIV-S | trimerized H5 |

The relevant amino acid sequences of isolated scFv binders and their alignment are shown in Figure 19, The sequencing revealed that clones 3, 9 and 12 were identical as well as clones 13 and 14 limiting the total number of unique scFv clones to eleven.

### EXAMPLE 4- Screening of antibody clones

### Microlitre plate (MTP) production of monoclonal soluble antibody fragments for screening.

*E. coli* colonies carrying Phagemid encoding single scFv antibody clones were picked and transferred into 96-well U-bottom polypropylene microtiter plates (Greiner Bio-One GmbH, Frickenhausen, Germany) containing 120 µL/well 2xYT-GA using and incubated at 37°C and 850 rpm in MTP-shaker for 6 to 8 hours. Thereafter, 20 µL of each well were transferred to a new well containing 180 µL 2xYT-GA and incubated for 2 hours at 37°C and 850 rpm. Cells were harvested by centrifugation at 3,200 xg, RT for 10 min. Supernatant was discarded and pellets were resuspended in 200µL per well 2xYT-A supplemented with 50 µM isopropylthiogalactoside (IPTG) to induce production of scFv antibody fragments. Production was performed at 30°C and 850 rpm over night. Supernatants were harvested by centrifugation at 3,200 x g at 4°C for 10 min and used in the screening ELISA.

### ELISA for screening and characterization of target binding scFv.

Single chain clones were checked for their binding specificity regarding monomeric H5 and lysozyme immobilized to NUNC MAxisorp plates. The trimeric H5 antigen was immobilized to ® M280 Streptavidin beads (Invitrogen) and the monomeric H5 target was immobilized to ® M270 Carboxylic Acid (Invitrogen). Both target beads were incubated using an over-head rotator. Beads were blocked with 2%M-PBST for 2 h at RT. 1µL target beads was used per well. Supernatants containing soluble monoclonal scFv antibody fragments were incubated 1:2 diluted in 2%M-PBST for 1 h at RT. After washing using an ELISA washer (Hydroflex, TECAN) bound scFv were stained with the mouse anti-myc-tag monoclonal antibody (mAb) 9E10 and a secondary goat anti-mouse-IgG antibody horse radish peroxidase (HRP) conjugate. For detection a two-step detection method was applied consisting of mouse monoclonal anti-cMyc 9E10 antibody detecting the myc-tag of the single chains and Sigma A0168 anti-mouse-Fc antibody conjugated to horse-radish-peroxidase (HRP). Color reaction was developed using 3,3',5,5'-tetramethylbenzidine (TMB, Sigma, Munich, Germany) Substrate solution: 20 volumes of solution A (30 mM potassium citrate, 0.5 mM citric acid, pH4.1) and 0.5 volumes of solution B (10 mM TMB in 10% (v/v) acetone, 89% (v/v) ethanol, 1% (v/v) of 30% H₂O₂). Stop solution: 1 volume 1 N H₂SO₄. Absorption was measured at 450nm versus a reference wavelength of 620nm.

Only those binders specifically binding to both H5 targets (monomer human and trimer avian) were chosen for further experiments. 11 single chain antibody clones showed specific binding to both H5 targets and were chosen for further investigation.

### EXAMPLE 6 - Construction, production and purification of bivalent scFv-Fc antibodies

Single chain DNA was cloned into two different expression vectors to allow eukaryotic expression of two different single-chain Fc-fusions. Using pCMX2.5-hlgG1-XP vector (Figure 2B) allows for the production of two single chain fragments fused to a human Fc-part of an antibody (IgG1-subclass). Using pCMV2.5-mlgG1-XP vector (Figure 2A) allows for the production of two single chain fragments fused to a murine Fc-part of an antibody (IgG1-subclass).

In other words, scFv gene fragments were subcloned into mammalian expression vectors to obtain bivalent scFv-Fc antibodies that resemble in many properties entire IgGs. Each scFv antibody clone was converted into two different scFv-Fc constructs containing the human IgG1-Fc or the mouse IgG1-Fc moiety, respectively. ScFv gene fragments were cloned for this purpose into the mammalian expression vectors pCMV-mlgG1-Fc XP (Hust et al, 2010 A human scFv antibody generation pipeline for proteome research J Biotechnol.) and pCMX2.5-human IgG1-Fc and pCSE 2.5 mlgG2cFc using *N*coI and *Not*I (NEB) to obtain fusion constructs of scFv with mouse IgG1-Fc, human IgG1Fc or mouse IgG2cFc, respectively. Production was performed either in adherent HEK293T cells or HEK293-6E suspension cells. Transient transfection was performed with polyethylenimide (PEI - PolyPlus, FR) as transfection agent. Transfection in HEK293T cells was done in DMEM / 8% FCS / 1% (v/v) penicillin/streptomycin. After 24 h medium was changed to DMEM / 4% low IgG-FCS / 1% (v/v) penicillin/streptomycin. Production supernatant was saved every day including a complete medium exchange for up to 10 days. Transfection in HEK293-6E cells was performed in serum free culture medium as described by Schirrmann and Büssow in 2010. 48 h after transfection 0.5 volumes of culture medium supplemented with tryptone N1 (final concentration 0.5% (w/v), Organotechnie S.A.S., La Courneuve, France) was added (Pham et al. 2005).

Cell culture supernatants were purified via Protein A columns Bio-Scale Mini UNOsphere SUPrA affinity chromatography cartridges, 1 mL (#732-4200 FIRMA) and Bio-Scale Mini Bio-Gel P-6 Desalting Cartridges #732-5304) using Profinia™ (BioRad) according to manufacturer's protocols. Due to the low affinity of mouse-IgG1-Fc to protein A the purification of the scFv-mIgG1 Fc antibodies was performed under high salt conditions (NaCl added to cell culture supernatant to final 1 M; binding buffer 2 M NaCl, 2.7 mM KCI, 4.3 mM Na₂HPO₄, 8.1 mM KH₂PO₄).

### EXAMPLE 7 - Antigen enzyme linked immunosorbent assay (ELISA) with scFv-Fc antibodies.

If not indicated otherwise, all monomeric H5 and control antigens (lysozyme, BSA) were immobilized to Maxisorp plates (NUNC) in 50 mM carbonate buffer pH 9.6 or PBS, respectively. Trimeric H5 target was always immobilized to NUNC polysorp strepatavidin plates or M280 streptavidin magnetic beads. Capture antibodies were always coated in PBS (4°C over night). All wells were blocked with 2%M-PBS for 1 h at RT. Secondary antibodies used for scFv-Fc detection were either goat-anti-human-IgGFc (Sigma A0170) or goat-anti-mouse-IgGFcγ-specific antibody HRP conjugates (Dianova, #115-035-071). Color reaction with TMB and measurement was performed as described above. For the virus capture ELISA scFv-hIgG1Fc antibodies were first immobilized to Maxisorp plates. After blocking and incubation with virus preparations, bound virus was detected by scFv-mIgG1Fc or scFv-mlgG2cFc antibodies as indicated and the appropriate secondary anti-mouse IgG-Fc antibody conjugate (goat-anti-mouse-IgGFcγ-specific antibody HRP conjugates (Dianova, #115-035-071).

### EXAMPLE 7 - Western Immunoblot

Recombinant monomeric H5 antigen was separated by a 10% SDS-PAGE (Sambrook et al., 1989) and blotted onto polyvinylidene fluoride (PVDF) membrane. Bound scFv-hIgG1Fc antibodies were detected using a goat anti-human-IgG-Fc antibody conjugate and diaminobenzidine (DAB) solution consisting of 200µL DAB 25 mg/mL (Roth, Karlsruhe, Germany) and 1 µL 30% (v/v) H₂O₂ diluted in 10 mL substrate buffer (PBS, 0.02% (w/v) CoCl₂). Staining was performed for 5 minutes and stopped with water.

To investigate H5 specificity on membrane bound H5, expected to closely resemble the naive conformation in the virus, a panel of 14 different influenza viruses (11 of H5, 1 of H1, H2, H6 subtypes) was used in immunoperoxidase monolayer assay (IPMA). A summary of the reactivity spectrum of each individual clone is given. Unexpected low cross-reactivity with other H-subtypes and broad spectrum H5 binding was shown for all identified binders. Only some of the isolated binders showed cross-reactivity with H2 and H6 and no antibody cross-reacted with H1. One clone showed no cross-reactivity with H1, H2 or H6 but detected all 11 H5-subtypes tested. Thus, we found antibody clones which show high specificity to nine H5N1 variants and low cross reactivity to H1, H2, H6 and H7 influenza strains.

### EXAMPLE 8 - Immunoperoxydase monolayer assay (IPMA)

IPMA assays were performed using STOMA 24 cells (FLI strain "recombinant ST606"), a swine testis cell line. STOMA24 cells were infected with influenza virus strains representing a broad range of H5 variants. In the wells of a 96 microtitration plate, 100 µl STOMA 24 cell suspension for obtaining a confluent cell layer in serum-free medium supplemented with antibiotics (G418, hypomycin, doxycyclin) was incubated per well with 100 µL virus suspension (virus supernatant from embryonated egg) for 2 days at 37°C. The ratio of STOMA 24 cells and virus load was adjusted for each virus to obtain approximately 5% cell infection. The supernatant was removed, the cells washed with 50 µl PBS phosphate buffer and then the cell and virus proteins (HA) fixed to the well bottom using methanol/aceton (40/60), followed by further washings. The virus proteins were then incubated with recombinant antibody clone supernatant, diluted in washing buffer (50 µl/well) for 1 hour at room temperature, followed by further washings and colour development (15 to 30 min at 37°C) using a HRP-conjugated secondary antibody (goat anti-human Fc from Sigma, Hamburg, DE), H₂O₂ (1µL 30% H₂O₂ per ml) and ready-to-use AEC (3-amino-9-ethylcarbazole) solution in 50 mM/L sodium-acetate stabilizing buffer, pH 5.0 (Blomol GmbH, Hamburg, DE). For hemagglutinins from highly pathogenic strains (A/Canada goose/Germany/71/06 and A/chicken/Egypt/0879-NLQP/2008 H5N1) High Five insect cells expressing the recombinant H5 antigens were employed. Table 4 below shows the results with the isolated and scFv-human-Fc antibody clones.

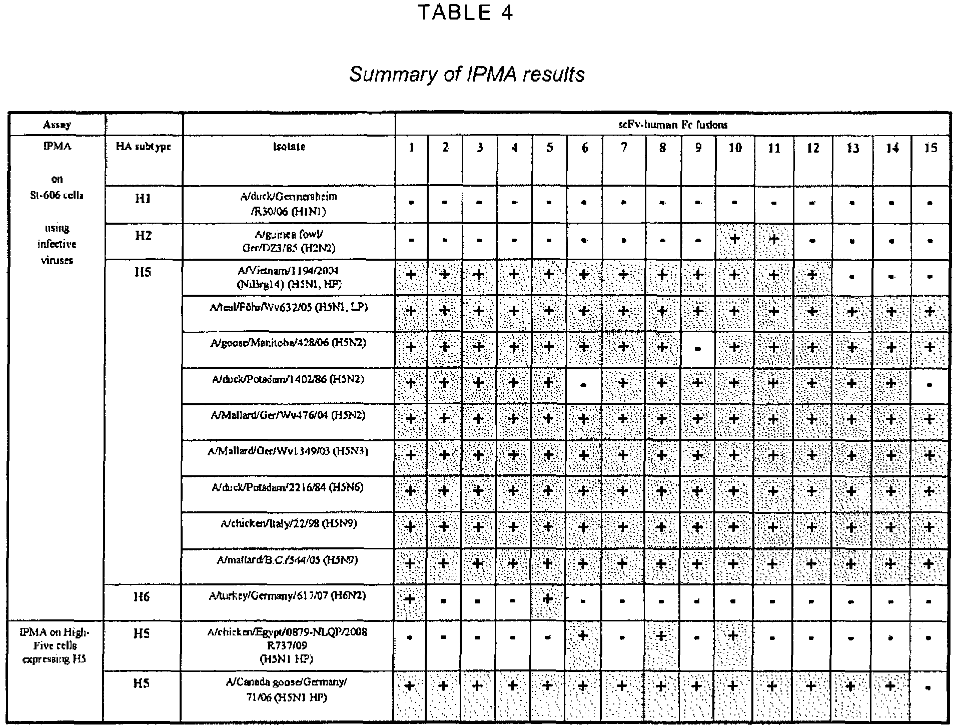

A large variety of influenza virus strains were used to represent the broadest range of H5 variants possible, Infective viruses were allowed to infect STOMA24 cells, a recombinant swine testis cell line (FLI declaration: CCLV-RIE 1199). Panning target trimerized H5 of A/Canada goose/Germany/71/06 H5N1 was presented by recombinant High Five cells expressing this H5 subtype. H5 of the influenza virus strain A/chicken/Egypt/0879-NLQP/2008-R737/09 likewise. Examples of IPMA results are given for two different clones in Figures 6, 7, and 8.

### EXAMPLE 10 - Epitope mapping

To determine the H5 epitope recognized by the isolated antibody fragments, consecutive 15mer peptides with an 3 amino acid overlap covering the entire H5 protein of A/Anhui/1/2005 (H5N1) were synthesized and spotted onto an amino-PEGylated cellulose membrane (AIMS Scientific Products GmbH, Wolfenbüttel, Germany) using SPOT synthesis technology. MIgG1Fc antibodies were then incubated with the peptide membrane and detected by appropriate goat anti-mouse-IgG-Fc antibody HRP conjugates. For results, please see Figures 17 and 19. In brief, the membrane was first blocked with 2% MPBST (PBS comprising 2% milk powder (Roth, DE; No. T1452) and 0.05% Tween® 20) for 1.5 hrs at RT and then incubated with serum/scFv-Fc (> 10 µg) in 2% MPBST for 2.5 hrs under shaking. Washings were done with PBS (8.5 NaCl, 1.34g Na₂HPO₄, 0.35 g NaH₂PO₄/1000 ml dH₂0) twice for 5 minutes, followed by an incubation with goat-anti-mouse IgG (Fc specific) (1:2000 in 2% MPBST) for 1.5 hrs. After incubation with the second antibody, two washing followed with TBS, 0.05% Tween® 20 and twice with CBS (8 g NaCl, 0.2 g KCI, 1.9 g citric acid, pH 7.0). The chromogenic reaction was done in a solution comprising CBS, 5 mM MgCl₂, 40µL M BCIP (5-Bromo-4-chloro-3-indolyl phosphate p-toluidine salt of Applichem, Gatersleben, DE), 60 µL MTT (Thiozolyl Blue = 3-[4,5-Dimethylthiazol-2-yl]-2,5-diphenyltetrazolium bromide of Sigma M5655).

### EXAMPLE 11 - Titration ELISA

To overcome issues of inconsistent virus immobilization and high background related to relatively low purity of virus preparations and high glycosylation of viruses, purified scFv-hIgG1Fc antibodies were first coated onto ELISA plates to specifically capture virus particles from solution. Captured viruses were then detected by serial concentrations of scFv-mIgG1Fc antibodies and an appropriate secondary anti-mouse IgG1Fc antibody conjugate.

Most of the scFv-Fc antibody clones showed half maximal antigen binding at a concentration of 150 ng/mL to H5 monomer directly immobilized onto Maxisorp plates. In other words, most binders showed half maximal binding to 500 ng/mL directly immobilized monomeric H5 at a concentration range from ∼250-500 ng/mL. Three clones (JM09, JM08 and JM01) showed half maximal antigen binding at very low concentration of 10-50 ng/mL (data not shown).

### EXAMPLE 12 - Immunoblot analyses

All binders further characterized were able to detect monomeric H5 and trimeric H5 in immunoblot analyses except clone JM07 which recognized monomeric H5 only. The binding specificity of some binders to other H5 antigens is exemplified. Results of immunoblottings for all isolated binders are summarized below. No cross-reactivity towards H7 was observed. Weaker binding of the antibodies to A/Vietnam/1197/2004 (H5N1) was likely due to lower amount of H5 in this recombinant virus compared to wild type influenza viruses (Harvey et al, 2010). The results of these immunoblot analyses are given in Table 5 below.

**TABLE 5**

| *Summary Western blot analyses* | | | | | | | | | | | | | | | | | |
|---|---|---|---|---|---|---|---|---|---|---|---|---|---|---|---|---|---|
| | | | **scFv-human Fc fusions** | | | | | | | | | | | | | | |
| **Target** | **H subtype** | **Origin of target** | **1** | **2** | **3** | **4** | **5** | **6** | **7** | **8** | **9** | **10** | **11** | **12** | **13** | **14** | **15** |
| A/Anhui/1/2005 | H5 | recombinant | + | + | + | + | + | + | + | + | + | + | + | + | + | + | + |
| A/Canada goose/Germany/71/06 | H5 | recombinant | + | + | + | + | + | + | - | + | + | + | + | + | + | + | + |
| A/teal/Föhr/Wv632/05 | H5 | Virus preparation | + | + | + | + | + | - | + | + | + | + | + | + | + | + | + |
| A/chicken/Egypt/0879-NLQP/2008-R737/09 | H5 | Virus preparation | - | - | - | - | - | + | - | + | - | - | - | - | - | - | - |
| A/Vietnam/1197/2004 (NIBRG14) | H5 | Virus preparation | - | - | - | - | - | - | - | - | - | - | - | - | - | - | - |
| A/chicken/ Germany/R28/03 | H7 | recombinant | - | - | - | - | - | - | - | - | - | - | - | - | - | - | - |

### EXAMPLE 13 - Epitope mapping using peptide SPOT membrane

Epitope mapping was performed by using a peptide spot membrane. 15mer peptides (185) derived from H5 A/Anhui/1/2005 were spotted onto a membrane. The peptides were designed with an 3 amino acid overlap.

In order to obtain an impression of the diversity of the HA proteins of influenza A virus isolates within subtype H, we compiled an alignment (MUSCLE, ref) of 26 complete HA protein sequences representing relevant clades within subtype H5. Sequences from Europe, Asia and North America were retrieved from Genbank. Substitution models were fitted according to the Bayesian information criterion using Modeltest (Posada 2008) as implemented in Topali (ref). Phylogenetic relationships based on the JTT+I+G model were estimated in a maximum likelihood (ML) framework using PhyML and the Approximate Likelihood Ratio Test (aLRT) as well as by Bayesian inference (BI). BI was conducted using MrBayes (ref). The BI analysis chain was sampled every 10^{th} of a total of 10.000 generations with a burn-in of 250 trees.

The analyses of the phylogenetic relationships of a few representative sequences of the HA protein of subtype H5 isolates results in a deeply furcated phylogenetic tree (Figure 15). Results of the ML analysis are shown, but BI yielded essentially similar totpologies. In principal, three clades can be distinguished which are composed of viruses isolated in the Americas, in Europe, and the HPAIV H5N1 group of Asian origin.

The minimal epitope identified by the antibody with the best specificity profile. with reactivity to all 11 H5 variants but no binding to H1, H2, H6 and H7, was identified to be RNVVW. The originally identified amino acid sequence after Spot membrane epitope mapping was FRNVVW. Virus A/chicken/Egypt/0879-NLQP/2008-R737/09 (H5N1 HP) shows amino acid substitution F144Y. Since this virus was detected by antibody JM08 as well in IPMA and immunoblot analyses we conclude that F144 is not part of the minimal epitope, In a 3D model of H5 generated with 3D-JIGSAW this epitope is located in close proximity to the receptor binding site and shows interference with this site. This region of the molecule has been described to be essential for binding of influenza viruses to sialic acid receptors prior endocytosis of the virus. (Skehel and Wiley, 2000). We further discovered that this conserved peptide epitope can be used as an antigenic peptide which elicits an immune response against highly variant H5N1 virus.

Extensive H5 sequence alignment revealed the epitope to be highly conserved and H5 specific. We performed H5 amino acid data base research using the public data base Influenza Virus Resource and aligned near full length HA amino acid sequences from H5N1 viruses from Asia (1294), Europe (253) and Africa (179) (suppl.). We identified 25 mutations within our five amino acid epitope which is a coverage of 98.55% of all analysed sequences. The found minimal epitope is also conserved in Egyptian variants which are known to be highly diverse due to a high mutation rate (65 mutated sequences). The disclosed five amino acid epitope is highly conserved among nearly all analysed sequences.

### EXAMPLE 14 - Microneutralization assays

To check for neutralizing activity of identified antibody fragments we performed microneutralization assays. We seeded 4-5 x 10⁴ MDCK+ cells per well in 96 well plates and allowed those to settle over night prior to use and infection. For each assay viruses with 100KID 50 were used to infect confluent cell layer.

ScFv-mouselgG2cFc fusions were pre-incubated with viruses for 1h at 37°C. Prior addition of pre-incubated virus-antibody mixture cells were washed twice in PBS to remove FCS. The cells were trypsinated a) prior to the incubation with the virus or b) 6h after addition of virus-antibody mixture. Assays were performed in quadruplets. Neutralization efficacy was determined after 72 h of incubation at 37°C. The results have been summarized in Table 5 below

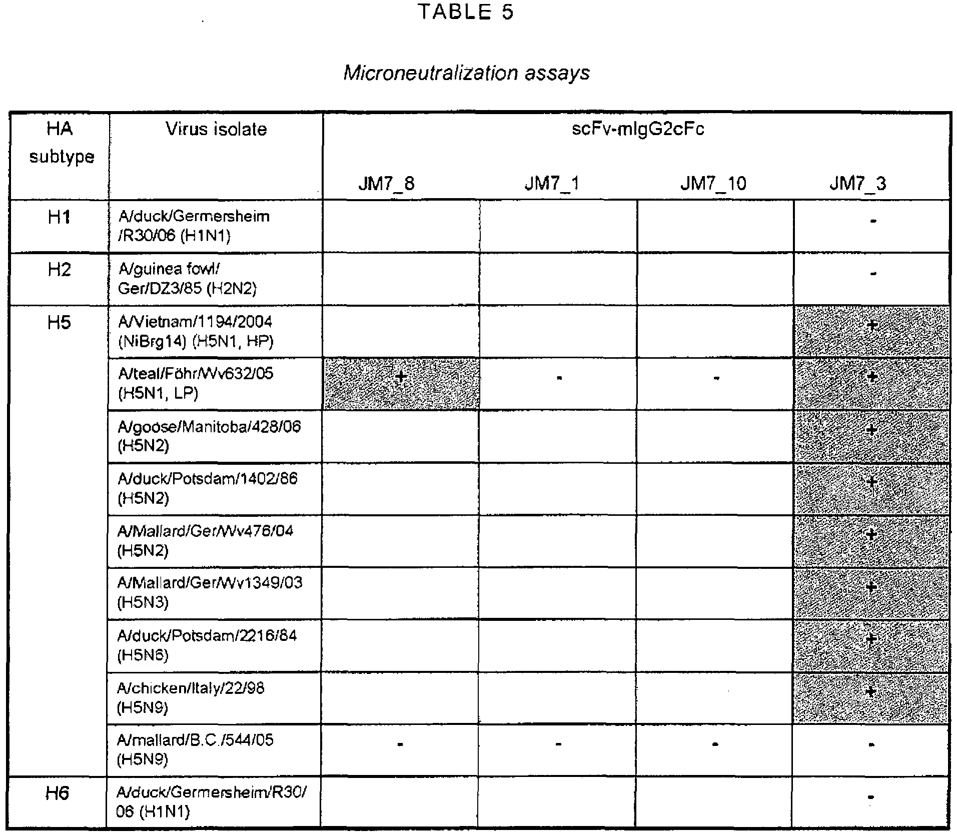

The results show that JM7_3 neutralizes all viruses checked except for A/mallard/B.C./544/05 until a final antibody concentration of 12.7ng/mL (1.9ng per well; end of antibody titration series). For this antibody neutralizing activity was not dependant on assay procedure.

JM7_8 did not show neutralizing effect on A/teal/Föhr/WV632/05 when trypsin was added prior incubation with viruses/antibody mixture (procedure a). JM7_8 however well neutralized A/teal/Föhr/Wv 632/05 until a final antibody concentration of 12.7ng/mL (1.9ng/well; end point of antibody titration series) when changing the assay procedure to a 6h delayed addition of trypsin (procedure b). Neutralizing activity of this antibody was not tested on other H5 influenza viruses.

For JM7_8 results from adapted microneutralization assay with 6h delay in addition of trypsin are shown. For all other antibodies microneutralization assay in standardized form (trypsin, virus and antibody added at the same time) was performed. Results from JM7_3 with A/teal/Föhr/Wv632/05 and A/mallard/B.C./544/05 were repeated under adapted microneutralization protocol and proven to be identical.

In summary, neutralization activity of antibody JM7_8 could be shown for A/teal/Föhr/Wv632/05 in microneutralization assay when trypsin is added with a 6 hours delay. As was shown in stability assays trypsin completely degrades JM7_8 scFvmIgG2c antibody within one hour at 37°C. JM7_8 neutralizes viruses at 100KID 50 at least with an antibody amount of 1.9ng/well (12.7ng/mL). It can be assumed that neutralization for other viruses of H5 subtype listed will be similar to neutralization of JM7_3.

JM7_3 neutralizes all viruses of H5 subtype except for A/mallard/B.C./544/05 at 100 KID 50 with an antibody amount of lower than 1.9ng/well (12.7ng/mL). For virus A/teal/Föhr/WV632/05 we did not observe any difference in neutralization activity between standard procedure for microneutralization assay and adapted procedure with 6 hour delay in trypsin addition. As could be shown in stability assays JM7_3 is less degraded by trypsin than JM7_8. Viral epitopes bound by JM7_8 and JM7_3 are therefore interesting epitopes for vaccine design.

### EXAMPLE 15 - TEM

Binding of isolated antibodies to whole viruses and not only to recombinant hemagglutinin was shown via TEM pictures (negative contrast staining). Protein surface structure of the viruses could be clearly shown (Fig 20 a-c). Background binding of 15nm protein-A-gold to the sample was very low (Fig. 20 d). Antibodies bound very specific to viral surfaces without any detectable background binding (Fig e-k). Amount of detected gold-particles per picture is much higher for samples with isolated anti-H5 antibodies compared to samples with 15 nm protein-A-gold alone.

## Claims

1. A receptor molecule or antibody or antibody fragment against an antigenic determinant which comprises the following amino acid sequence
SEQ ID NO: 3 RNVVW

2. The receptor, antibody or antibody fragment of claim 1, which antigenic determinant takes a spatial structure corresponding to an antigenic determinant that comprises the amino acid sequence SEQ ID NO: 3 corresponding to residues 145 to 149 in a head domain of a H5 hemagglutinin molecule.

3. The receptor, antibody or antibody fragment of claim 1 or claim 2, wherein said spatial structure is based on the amino acid sequence SEQ ID NO: 2 (F)RNVVW, wherein (F) is optional.

4. The receptor, antibody or antibody fragment of claim 3, wherein said spatial structure belongs to a molecule of H5 hemagglutinin serotype.

5. The receptor, antibody or antibody fragment of any claim 1 to 4, comprising the variable region of the Heavy Chain and the variable region of the Light Chain present in following amino acid sequence
SEQ ID NO: 4

6. Receptor, antibody or antibody fragment as claimed in any claim 1 to 5 for use in a method of passive vaccination of an animal inflicted of influenza, notably influenza caused by a virus of subtype H5N1.

7. Use of a receptor, antibody or antibody fragment as claimed in any claim 1 to 5 for in vitro diagnosis or detection of H5 influenza virus.

## Patentansprüche

1. Rezeptormolekül oder Antikörper oder Antikörperfragment gegen eine antigene Determinante, welche die folgende Aminosäuresequenz umfasst
SEQ ID NO: 3 RNVVW

2. Rezeptor, Antikörper oder Antikörperfragment gemäß Anspruch 1, dessen antigene Determinante eine Raumstruktur einnimmt, entsprechend einer antigenen Determinante, umfassend die Aminosäuresequenz SEQ ID NO: 3, entsprechend den Resten 145 bis 149 in einer Kopfdomäne eines H5-Hemagglutinin-Moleküls.

3. Rezeptor, Antikörper oder Antikörperfragment gemäß Anspruch 1 oder Anspruch 2, wobei die Raumstruktur auf der Aminosäuresequenz SEQ ID NO: 2 (F)RNVVW basiert, wobei (F) optional ist.

4. Rezeptor, Antikörper oder Antikörperfragment gemäß Anspruch 3, wobei die Raumstruktur zu einem Molekül mit dem H5-Hemagglutinin-Serotyp gehört.

5. Rezeptor, Antikörper oder Antikörperfragment gemäß irgendeinem Anspruch 1 bis 4, umfassend die variable Region der Schweren Kette und die variable Region der Leichten Kette, die enthalten sind in der folgenden Aminosäuresequenz
SEQ ID NO: 4

6. Rezeptor, Antikörper oder Antikörperfragment gemäß irgendeinem Anspruch 1 bis 5 zur Verwendung in einem Verfahren zur passiven Impfung eines von Influenza betroffenen Tieres, insbesondere Influenza, hervorgerufen durch einen Virus des Subtyps H5N1.

7. Verwendung eines Rezeptors, Antikörpers oder Antikörperfragments aus irgendeinem Anspruch 1 bis 5 für in vitro-Diagnose oder Bestimmung von H5Influenza-Virus.

## Revendications

1. Molécule réceptrice, anticorps ou fragment d'anticorps contre un déterminant antigénique qui comprend la séquence d'acides aminés suivante
SEQ ID NO: 3 RNVVW

2. Récepteur, anticorps ou fragment d'anticorps selon la revendication 1, dont le déterminant antigénique prend une structure spatiale correspondant à un déterminant antigénique qui comprend la séquence d'acides aminés SEQ ID NO : 3 correspondant aux résidus 145 à 149 dans un domaine de tête d'une molécule H5-hémagglutinine.

3. Récepteur, anticorps ou fragment d'anticorps selon la revendication 1 ou la revendication 2, dans lequel ladite structure spatiale est basée sur la séquence d'acides aminés SEQ ID NO : 2 (F)RNVVW, où (F) est optionnel.

4. Récepteur, anticorps ou fragment d'anticorps selon la revendication 3, dans lequel ladite structure spatiale appartient à une molécule de sérotype H5-hémagglutinine.

5. Récepteur, anticorps ou fragment d'anticorps selon une quelconque revendication 1 à 4, comprenant la région variable de la Chaîne Lourde et la région variable de la Chaîne Légère présente dans la séquence d'acides aminés suivante
SEQ ID NO: 4

6. Récepteur, anticorps ou fragment d'anticorps selon une quelconque revendication 1 à 5 pour l'utilisation dans un procédé de vaccination passive d'un animal infligé d'influenza, en particulier d'influenza causée par un virus de sous-type H5N1.

7. Utilisation d'un récepteur, anticorps ou fragment d'anticorps revendiqué dans une quelconque revendication 1 à 5 pour un diagnostic in vitro ou la détection du virus influenza H5.


### SEQUENCE LISTING

<110> mAB factory GmbH
<120> UNIVERSAL VACCINE AGAINST INFLUENZA H5N1 VIRUSES, MEDICAMENT AND TREATMENT OF H5N1 VIRAL INFECTIONS
<130> GP100171PC00
<140> not known
   <141> 2011-12-02
<160> 41
<170> PatentIn version 3.3
<210> 1
   <211> 21
   <212> PRT
   <213> Influenza A virus
<400> 1
   Phe Arg Asn Val Val Trp Leu Ile Lys Lys Asn Asn Thr Tyr Pro Thr
   1               5                   10                  15
   Ile Lys Arg Ser Tyr
               20
<210> 2
   <211> 6
   <212> PRT
   <213> Influenza A virus
<400> 2
   Phe Arg Asn Val Val Trp
   1               5
<210> 3
   <211> 5
   <212> PRT
   <213> Influenza A virus
<400> 3
   Arg Asn Val Val Trp
   1 5
<210> 4
   <211> 481
   <212> PRT
   <213> Artificial
<220>
   <223> Antibody sequence
<400> 4
<210> 5
   <211> 760
   <212> DNA
   <213> Chicken
<400> 5
   ccatggccgc cgtgacgttg gacgagtccg ggggcggcct ccagaagccc ggaggagggc      60
   tcagcctcgt ctgcaaggcc tccgggttcc ccttcagcag ttacagcatg cagtgggtgc     120
   gacaggcacc cggcaagggg ctggaatggg tcgcaggtat tggcaatgct ggtagttaca     180
   caggatatgg ggcggcggtg aagggccgtg ccaccatctc gagggacaac gggcagagca     240
   cagtgaggct gcagctgaac aacctcaggg ctgaggacac cggcacctac tactgcgcca     300
   aaagtgctta tgctggtagt tgtgctggtt gtgttgatag catcgacgca tggggccacg     360
   ggaccgaagt catggtctcc tccgggagtg catccgcccc aaagcttgaa gaaggtgaat     420
   tttcagaagc acgcgtagcg ctgactcagc cgtcctcggt gtcagcgaac ccgggagaaa     480
   ccgtcaagat cacctgctcc ggggatgaca gctggtatgg ctggtatcag cagaagtcac     540
   ctggcagtgc ccctgtcact gtgatctatt acaacaacaa cagaccctcg gacatccctt     600
   tacgattctc cggttccaca tctggctcca caagcacatt aaccatcact ggggtccaag     660
   ccgacgacga ggctgtctat ttctgtggga gctgggacag gagcagtggt gctggtatat     720
   ttggggccgg gacaaccctg accgtcctag gtgcggccgc                           760
<210> 6
   <211> 758
   <212> DNA
   <213> Artificial
<220>
   <223> DNA encoding scFv
<400> 6
   atggccgccg tgacgttgga cgagtccggg ggcggcctcc agacgcccgg aggagggctc     60
   agtctcgtct gcaaggcctc cgggttcatc ttcagtgacc gtggcatgca ctgggtgcga    120
   caggcgcccg gcaaggggct ggagtgggtc ggtggtattg gcagcagtgg tagtagcaca    180
   tggtacgggg cggcggtgaa gggccgtgcc acaatctcga gggacaacgg gcagagcaca    240
   gtgaggctgc agctgaacaa cctcagggct gaggacaccg gcacctacta ctgcgccaaa    300
   gatgctagta gtggtggtgg tggtagtcct tatggtgttg ctagaatcga cgcatggggc    360
   cacgggaccg aagtcatggt ctcctccggg agtgcatccg ccccaaagct tgaagaaggt    420
   gaattttcag aagcacgcgt agcgctgact cagccgtcct cggtgtcagc aaacctggga    480
   ggaaccgtcg agatcacctg ctccgggggt ggtggcagct atggctggta ccagcagaag    540
   tctcctggca gtgcccctgt cactgtaatc tattacaacg acaagagacc ctcggacatc    600
   ccttcacgat tctccggttc cgcatccggc tccacagcca cattaaccat cactggggtc    660
   caagtcgggg acgaggctgt ctattactgt gcgagtgcag acagcagtgc tgctgcattt    720
   ggggccggga caaccctgac cgtcctaggt gcggccgc                            758
<210> 7
   <211> 252
   <212> PRT
   <213> Artificial
<220>
   <223> scFv antibody sequence
<400> 7
   Met Ala Ala Val Thr Leu Asp Glu Ser Gly Gly Gly Leu Gln Thr Pro
   1               5                   10                  15
   Gly Gly Gly Leu Ser Leu Val Cys Lys Ala Ser Gly Phe Ile Phe Ser
               20                  25                  30
   Asp Arg Gly Met His Trp Val Arg Gln Ala Pro Gly Lys Gly Leu Glu
           35                  40                  45
   Trp Val Gly Gly Ile Gly Ser Ser Gly Ser Ser Thr Trp Tyr Gly Ala
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Thr Tyr
                   85                  90                  95
   Tyr Cys Ala Lys Asp Ala Ser Ser Gly Gly Gly Gly Ser Pro Tyr Gly
               100                 105                 110
   Val Ala Arg Ile Asp Ala Trp Gly His Gly Thr Glu Val Met Val Ser
           115                 120                 125
   Ser Gly Ser Ala Ser Ala Pro Lys Leu Glu Glu Gly Glu Phe Ser Glu
       130                 135                 140
   Ala Arg Val Ala Leu Thr Gln Pro Ser Ser Val Ser Ala Asn Leu Gly
   145                 150                 155                 160
   Gly Thr Val Glu Ile Thr Cys Ser Gly Gly Gly Gly Ser Tyr Gly Trp
                   165                 170                 175
   Tyr Gln Gln Lys Ser Pro Gly Ser Ala Pro Val Thr Val Ile Tyr Tyr
               180                 185                 190
   Asn Asp Lys Arg Pro Ser Asp Ile Pro Ser Arg Phe Ser Gly Ser Ala
   195 200 205
   Ser Gly Ser Thr Ala Thr Leu Thr Ile Thr Gly Val Gln Val Gly Asp
   210 215 220
   Glu Ala Val Tyr Tyr Cys Ala Ser Ala Asp Ser Ser Ala Ala Ala Phe
   225 230 235 240
   Gly Ala Gly Thr Thr Leu Thr Val Leu Gly Ala Ala
   245 250
<210> 8
   <211> 9
   <212> PRT
   <213> Influenza A virus
<400> 8
   Val Pro Lys Ile Ala Thr Arg Ser Lys
   1 5
<210> 9
   <211> 15
   <212> PRT
   <213> Influenza A virus
<400> 9
   Gly Thr Ser Thr Leu Asn Gln Arg Leu Val Pro Lys Ile Ala Thr
   1 5 10 15
<210> 10
   <211> 15
   <212> PRT
   <213> Influenza A virus
<400> 10
   Thr Leu Asn Gln Arg Leu Val Pro Lys Ile Ala Thr Arg Ser Lys
   1 5 10 15
<210> 11
   <211> 15
   <212> PRT
   <213> Influenza A virus
<400> 11
   Gln Arg Leu Val Pro Lys Ile Ala Thr Arg Ser Lys Val Asn Gly
   1 5 10 15
<210> 12
   <211> 15
   <212> PRT
   <213> Influenza A virus <400> 12
<210> 13
   <211> 16
   <212> PRT
   <213> Influenza A virus <400> 13
<210> 14
   <211> 760
   <212> DNA
   <213> Artificial
<220>
   <223> DNA encoding scFv
<400> 14
   ccatggccgc cgtgacgttg gacgagtccg ggggcggcct ccagacgccc ggaggagcgc 60
   tcagtctcgt ctgcaaggcc tccgggttca tcttcagtga ccgtggcatg cactgggtgc 120
   gacaggcgcc cggcaagggg ctggagtggg tcggtggtat tggcagcagt ggtagtagca 180
   catggtacgg ggcggcggtg aagggccgtg ccacaatctc gagggacaac gggcagagca 240
   cagtgaggct gcagctgaac aacctcaggg ctgaggacac cggcacctac tactgcgcca 300
   aagatgctag tagtggtggt ggtggtagtc cttatggtgt tgctagaatc gacgcatggg 360
   gccacgggac cgaagttatg gtctcctccg ggagtgcatc cgccccaaag cttgaagaag 420
   gtgaattttc agaagcacgc gtagcgctga ctcagccgtc ctcggtgtca gcaaacctgg 480
   gaggaaccgt cgagatcacc tgctccggga gttggggcag ctatggctgg taccagcaga 540
   gggcacctgg cagtgcccct gtcactgtga tctatgaaac caccaagaga ccctcgaaca 600
   tcccttcacg attctccggt tccaaatctg gctccacaca cacattaacc atcactgggg 660
   tccaagccga ggacgaggct gtctattact gtgggagtgc agacagcagc gtcggtatat 720
   ttggggccgg gacaaccctg accgtcctag gtgcggccgc 760
<210> 15
   <211> 252
   <212> PRT
   <213> Artificial
<220>
   <223> scFv antibody sequence
<400> 15
   Met Ala Ala Val Thr Leu Asp Glu Ser Gly Gly Gly Leu Gln Thr Pro
   1 5 10 15
   Gly Gly Ala Leu Ser Leu Val Cys Lys Ala Ser Gly Phe Ile Phe Ser
   20 25 30
   Asp Arg Gly Met His Trp Val Arg Gln Ala Pro Gly Lys Gly Leu Glu
   35 40 45
   Trp Val Gly Gly Ile Gly Ser Ser Gly Ser Ser Thr Trp Tyr Gly Ala
   50 55 60
   Ala Val Lys Gly Arg Ala Thr Ile Ser Arg Asp Asn Gly Gln Ser Thr
   65 70 75 80
   Val Arg Leu Gln Leu Asn Asn Leu Arg Ala Glu Asp Thr Gly Thr Tyr
   85 90 95
   Tyr Cys Ala Lys Asp Ala Ser Ser Gly Gly Gly Gly Ser Pro Tyr Gly
   100 105 110
   Val Ala Arg Ile Asp Ala Trp Gly His Gly Thr Glu Val Met Val Ser
   115 120 125
   Ser Gly Ser Ala Ser Ala Pro Lys Leu Glu Glu Gly Glu Phe Ser Glu
   130 135 140
   Ala Arg Val Ala Leu Thr Gln Pro Ser Ser Val Ser Ala Asn Leu Gly
   145 150 155 160
   Gly Thr Val Glu Ile Thr Cys Ser Gly Ser Trp Gly Ser Tyr Gly Trp
   165 170 175
   Tyr Gln Gln Arg Ala Pro Gly Ser Ala Pro Val Thr Val Ile Tyr Glu
   180 185 190
   Thr Thr Lys Arg Pro Ser Asn Ile Pro Ser Arg Phe Ser Gly Ser Lys
   195 200 205
   Ser Gly Ser Thr His Thr Leu Thr Ile Thr Gly Val Gln Ala Glu Asp
   210 215 220
   Glu Ala Val Tyr Tyr Cys Gly Ser Ala Asp Ser Ser Val Gly Ile Phe
   225 230 235 240
   Gly Ala Gly Thr Thr Leu Thr Val Leu Ala Ala Ala
   245 250
<210> 16
   <211> 779
   <212> DNA
   <213> Artificial
<220>
   <223> DNA encoding scFv
<400> 16
   atggccgccg tgacgttgga cgagtccggg ggcggcctcc agacgcccgg aggagcgctc      60
   agcctcgtct gcaaggcctc cgggttctct atcagcggtt acaacatgaa ctgggtgcga     120
   caggcgcccg gcaaggggct ggagtgggtc gctgaaatta gcgatgatgg tagttacaca     180
   tactacgggg cggcggtgaa gggccgtgcc accatctcga gggacaacgg gcagagcaca     240
   gtgaggctgc agttgaacaa cctcagggct gaggacaccg gcatctacta ctgcgccaaa     300
   tctgctggta atgctggtgg tagtggttat agttatactg gtttgatcga cgcatggggc     360
   cacgggaccg aagtcatggt ctcctccggg agtgcatccg ccccaaagct tgaagaaggt     420
   gaattttcag aagcacgcgt agcgctgact cagccgtcct cggtgtcagc aaacccggga     480
   gaaaccgtca agatcacctg ctccgggggt ggcagctatg ctggaagtta ctattatggc     540
   tggtaccagc agaagtcacc tggcagtgcc cctgtcactg tgatctatag caacgacaag     600
   agaccctcgg acatcccttc acgattctcc ggtgccctat ccggctccac agccacatta     660
   accatcactg gggtccaagc cgacgacgag gctgtctatt tctgtgggag tgcagacagc     720
   agcaatagtg ctggtatatt tggggccggg acaaccctga ccgtcctagg tgcggccgc      779
<210> 17
   <211> 259
   <212> PRT
   <213> Artificial
<220>
   <223> scFv
<400> 17
   Met Ala Ala Val Thr Leu Asp Glu Ser Gly Gly Gly Leu Gln Thr Pro
   1               5                   10                  15
   Gly Gly Ala Leu Ser Leu Val Cys Lys Ala Ser Gly Phe Ser Ile Ser
               20                  25                  30
   Gly Tyr Asn Met Asn Trp Val Arg Gln Ala Pro Gly Lys Gly Leu Glu
           35                  40                  45
   Trp Val Ala Glu Ile Ser Asp Asp Gly Ser Tyr Thr Tyr Tyr Gly Ala
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Ile Tyr
                   85                  90                  95
   Tyr Cys Ala Lys Ser Ala Gly Asn Ala Gly Gly Ser Gly Tyr Ser Tyr
               100                 105                 110
   Thr Gly Leu Ile Asp Ala Trp Gly His Gly Thr Glu Val Met Val Ser
           115                 120                 125
   Ser Gly Ser Ala Ser Ala Pro Lys Leu Glu Glu Gly Glu Phe Ser Glu
       130                 135                 140
   Ala Arg Val Ala Leu Thr Gln Pro Ser Ser Val Ser Ala Asn Pro Gly
   145                 150                 155                 160
   Glu Thr Val Lys Ile Thr Cys Ser Gly Gly Gly Ser Tyr Ala Gly Ser
                   165                 170                 175
   Tyr Tyr Tyr Gly Trp Tyr Gln Gln Lys Ser Pro Gly Ser Ala Pro Val
               180                 185                 190
   Thr Val Ile Tyr Ser Asn Asp Lys Arg Pro Ser Asp Ile Pro Ser Arg
           195                 200                 205
   Phe Ser Gly Ala Leu Ser Gly Ser Thr Ala Thr Leu Thr Ile Thr Gly
       210                 215                 220
   Val Gln Ala Asp Asp Glu Ala Val Tyr Phe Cys Gly Ser Ala Asp Ser
   225                 230                 235                 240
   Ser Asn Ser Ala Gly Ile Phe Gly Ala Gly Thr Thr Leu Thr Val Leu
                   245                 250                 255
   Gly Ala Ala
<210> 18
   <211> 763
   <212> DNA
   <213> Artificial
<220>
   <223> DNA encoding scFv
<400> 18
   ccatggccgc cgtgacgttg gacgagtccg ggggcggcct ccggacgccc ggaggagcgc 60
   tcagtctcgt ctgcaaggcc tccgggttca ccttcagtga ccgtggcatg cactgggtgc 120
   gacaggcgcc cggcaagggg ctggagtggg tcggtggtat tggcagcagt ggtagtagca 180
   catggtacgc gacggcggtg aagggccgtg ccaccatctc gaggaacaac gggcagagca 240
   cagtgaggct gcagctgaac agcctcaggg cagaggacac cggcacctac tattgcgcca 300
   aagatgctag cagtggtggt ggtggtagtc cttatggtgt tgctagaatc gacgcatggg 360
   gccacgggac cgaagtcatg gtctcctccg ggagtgcatc cgccccaaag cttgaagaag 420
   gtgaattttc agaagcacgc gtagcgctga ctcagccgtc ctcggtgtca gcgaacccgg 480
   gaggaaccgt caagatcacc tgctccggga gtagtggcag ctatggctgg tatcagcaga 540
   agtcacctgg cagtgcccct gtcactgtga tctattacaa cgacaagaga ccctcggaca 600
   tcccttcacg attctccggt tccaaatccg gctccacggg cacattaacc atcactgggg 660
   tccaagccga ggacgaggct gtctattact gtgggaatgc agacagcagc agtactgctc 720
   tatttggggc cgggacaacc ctgaccgtcc taggtgcggc cgc 763
<210> 19
   <211> 252
   <212> PRT
   <213> Artificial
<220>
   <223> scFv
<400> 19
   Met Ala Ala Val Thr Leu Asp Glu Ser Gly Gly Gly Leu Arg Thr Pro
   1               5                   10                  15
   Gly Gly Ala Leu Ser Leu Val Cys Lys Ala Ser Gly Phe Thr Phe Ser
               20                  25                  30
   Asp Arg Gly Met His Trp Val Arg Gln Ala Pro Gly Lys Gly Leu Glu
           35                  40                  45
   Trp Val Gly Gly Ile Gly Ser Ser Gly Ser Ser Thr Trp Tyr Ala Thr
       50                  55                  60
   Ala Val Lys Gly Arg Ala Thr Ile Ser Arg Asn Asn Gly Gln Ser Thr
   65                  70                  75                  80
   Val Arg Leu Gln Leu Asn Ser Leu Arg Ala Glu Asp Thr Gly Thr Tyr
                   85                  90                  95
   Tyr Cys Ala Lys Asp Ala Ser Ser Gly Gly Gly Gly Ser Pro Tyr Gly
               100                 105                 110
   Val Ala Arg Ile Asp Ala Trp Gly His Gly Thr Glu Val Met Val Ser
   115 120 125
   Ser Gly Ser Ala Ser Ala Pro Lys Leu Glu Glu Gly Glu Phe Ser Glu
   130 135 140
   Ala Arg Val Ala Leu Thr Gln Pro Ser Ser Val Ser Ala Asn Pro Gly
   145 150 155 160
   Gly Thr Val Lys Ile Thr Cys Ser Gly Ser Ser Gly Ser Tyr Gly Trp
   165 170 175
   Tyr Gln Gln Lys Ser Pro Gly Ser Ala Pro Val Thr Val Ile Tyr Tyr
   180 185 190
   Asn Asp Lys Arg Pro Ser Asp Ile Pro Ser Arg Phe Ser Gly Ser Lys
   195 200 205
   Ser Gly Ser Thr Gly Thr Leu Thr Ile Thr Gly Val Gln Ala Glu Asp
   210 215 220
   Glu Ala Val Tyr Tyr Cys Gly Asn Ala Asp Ser Ser Ser Thr Ala Leu
   225 230 235 240
   Phe Gly Ala Gly Thr Thr Leu Thr Val Leu Ala Ala
   245 250
<210> 20
   <211> 758
   <212> DNA
   <213> Artificial
<220>
   <223> DNA encoding scFv
<400> 20
   atggccgccg tgacgttgga cgagtccggg ggcggcctcc agacgcccgg aggagcgctc 60
   agtctcgtct gcaaggcctc cgggttcatc ttcagtgacc gtggcatgca ctgggtgcga 120
   caggcgcccg gcaaggggct ggagtgggtc ggtggtattg gcagcagtgg tagtagcaca 180
   tggtacgcga cggcggtgaa gggccgtgcc accatctcga gggacaacgg gcagagcaca 240
   gtgaggctgc agctgaacaa cctcagggct gaggacaccg gcacctacta ctgcgccaaa 300
   gatgctagta gtggtggtgg tggtagtcct tatggtgttg ctagaatcga cgcatggggc 360
   cacgggaccg aagtcatggt ctcctccggg agtgcatccg ccccaaagct tgaagaaggt 420
   gaattttcag aagcacgcgt agcgctgact cagccgtcct cggtgtcagc gaacccggga 480
   gaaaccgtcg agatcacctg ctccgggagt agtggcaggt atggctggta tcagcagaag 540
   tcacctggca gtgcccctgt cactctgatc tatagcaacg acaagagacc ctcggacatc 600
   ccttcacgat tctccggttc cgcatccggc tccacaaaca cattaaccat cactggggtc 660
   caagtcgagg acgaggctgt ctatttctgt gggagctacg acggcaatag cgatgtattt 720
   ggggccggga caaccctgac cgtcctaggt gcggccgc 758
<210> 21
   <211> 252
   <212> PRT
   <213> Artficial
<400> 21
   Met Ala Ala Val Thr Leu Asp Glu Ser Gly Gly Gly Leu Gln Thr Pro
   1               5                   10                  15
   Gly Gly Ala Leu Ser Leu Val Cys Lys Ala Ser Gly Phe Ile Phe Ser
               20                  25                  30
   Asp Arg Gly Met His Trp Val Arg Gln Ala Pro Gly Lys Gly Leu Glu
           35                  40                  45
   Trp Val Gly Gly Ile Gly Ser Ser Gly Ser Ser Thr Trp Tyr Ala Thr
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Thr Tyr
                   85                  90                  95
   Tyr Cys Ala Lys Asp Ala Ser Ser Gly Gly Gly Gly Ser Pro Tyr Gly
               100                 105                 110
   Val Ala Arg Ile Asp Ala Trp Gly His Gly Thr Glu Val Met Val Ser
           115                 120                 125
   Ser Gly Ser Ala Ser Ala Pro Lys Leu Glu Glu Gly Glu Phe Ser Glu
       130                 135                 140
   Ala Arg Val Ala Leu Thr Gln Pro Ser Ser Val Ser Ala Asn Pro Gly
   145                 150                 155                 160
   Glu Thr Val Glu Ile Thr Cys Ser Gly Ser Ser Gly Arg Tyr Gly Trp
                   165                 170                 175
   Tyr Gln Gln Lys Ser Pro Gly Ser Ala Pro Val Thr Leu Ile Tyr Ser
               180                 185                 190
   Asn Asp Lys Arg Pro Ser Asp Ile Pro Ser Arg Phe Ser Gly Ser Ala
   195 200 205
   Ser Gly Ser Thr Asn Thr Leu Thr Ile Thr Gly Val Gln Val Glu Asp
   210 215 220
   Glu Ala Val Tyr Phe Cys Gly Ser Tyr Asp Gly Asn Ser Asp Val Phe
   225 230 235 240
   Gly Ala Gly Thr Thr Leu Thr Val Leu Ala Ala Ala
   245 250
<210> 22
   <211> 749
   <212> DNA
   <213> Artificial
<220>
   <223> DNA encoding scFv-clone 6
<400> 22
   atggccgccg tgacgttgga cgagtccggg ggcggcctcc agacgcccgg aggagcgctc      60
   agcctcgtct gcaagggctc cgggttctct gtcggcagtt atgccatgtt ctgggtgcga     120
   caggcgcccg gcaaaggact ggagtatgtc gctggtattg agaatgatgg tagtagtaca     180
   tggtacgcga cggcggtgaa gggccgtgcc accatctcga gggacaacgg gcagagcaca     240
   gtgaggctgc agctgaacaa cctcagggct gaggacaccg gcacctacta ctgtgccaga     300
   ggtgatggtc ctgacagttt cgacgcatgg ggccacggga ccgaagtcat ggtctcctcc     360
   gggagtgcat ccgccccaaa gcttgaagaa ggtgaatttt cagaagcacg cgtagcgctg     420
   actcagccgt cctcggtgtc agcaaaccca ggagaaaccg tcaagatcac ctgctccggg     480
   ggtagtagct atgctggaag ttactattat ggctggtacc agcagaagtc tcctggcagt     540
   gcccctgtca ctgtgatcta tagcaacaac cagagaccct cggacatccc ttcacgattc     600
   tccggttcca catccggctc cacaagcaca ttaaccatca ctggggtcca agccgacgac     660
   gaggctgtct atcactgtgg tgcctacgac agcacttatc ttggtatatt tggggccggg     720
   acaaccctga ccgtcctagg tgcggccgc                                       749
<210> 23
   <211> 249
   <212> PRT
   <213> Artificial
<220>
   <223> aa of scFv clone 6
<400> 23
   Met Ala Ala Val Thr Leu Asp Glu Ser Gly Gly Gly Leu Gln Thr Pro
   1               5                   10                  15
   Gly Gly Ala Leu Ser Leu Val Cys Lys Gly Ser Gly Phe Ser Val Gly
               20                  25                  30
   Ser Tyr Ala Met Phe Trp Val Arg Gln Ala Pro Gly Lys Gly Leu Glu
           35                  40                  45
   Tyr Val Ala Gly Ile Glu Asn Asp Gly Ser Ser Thr Trp Tyr Ala Thr
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Thr Tyr
                   85                  90                  95
   Tyr Cys Ala Arg Gly Asp Gly Pro Asp Ser Phe Asp Ala Trp Gly His
               100                 105                 110
   Gly Thr Glu Val Met Val Ser Ser Gly Ser Ala Ser Ala Pro Lys Leu
           115                 120                 125
   Glu Glu Gly Glu Phe Ser Glu Ala Arg Val Ala Leu Thr Gln Pro Ser
       130                 135                 140
   Ser Val Ser Ala Asn Pro Gly Glu Thr Val Lys Ile Thr Cys Ser Gly
   145                 150                 155                 160
   Gly Ser Ser Tyr Ala Gly Ser Tyr Tyr Tyr Gly Trp Tyr Gln Gln Lys
                   165                 170                 175
   Ser Pro Gly Ser Ala Pro Val Thr Val Ile Tyr Ser Asn Asn Gln Arg
               180                 185                 190
   Pro Ser Asp Ile Pro Ser Arg Phe Ser Gly Ser Thr Ser Gly Ser Thr
           195                 200                 205
   Ser Thr Leu Thr Ile Thr Gly Val Gln Ala Asp Asp Glu Ala Val Tyr
       210                 215                 220
   His Cys Gly Ala Tyr Asp Ser Thr Tyr Leu Gly Ile Phe Gly Ala Gly
   225                 230                 235                 240
   Thr Thr Leu Thr Val Leu Ala Ala Ala
                   245
<210> 24
   <211> 764
   <212> DNA
   <213> Artificial
<220>
   <223> DNA encoding scFv
<400> 24
   atggccgccg tgacgttgga cgagtccggg ggcggcctcc agacgcccgg aggagggttc      60
   agcctcgtct gcaaggcctc cgggttcacc ttcagcagtt acgccatgat gtgggtgcga     120
   caggcgcccg gcaagaggct ggagtgggtc gcgggtatta gtggtgctgg tagctacaca     180
   aactacgggg cggcggtgaa gggccgtgcc accatctcga gggacaacgg gcagagcaca     240
   gcgaggctgc agctgaacaa cctcagggct gaggacaccg gcacctactt ctgcgccaga     300
   agtagtggtt attgtggttt tagttgtgct gaatatatcg acgcatgggg ccacgggacc     360
   gaagtcatgg tctcctccgg gagtgcatcc gccccaaagc ttgaagaagg tgaattttca     420
   gaagcacgcg tagcgctgac tcagccgtcc tcggtgtcag caaacctggg aggaaccgtc     480
   aagatcacct gctccggggg tggcagctat tatggctggt accagcagaa ggcacctaac     540
   agtgcccctg tctctgtgat ctatgacaac accaacagac cctcgaacat cccttcacga     600
   ttctccggtt ccaaatccgg ctccacagcc acattaacca tcactggggt ccaagccgac     660
   gacgaggctg tctatttctg tgggagctac gacagcagta ctgatagtgg ttatgttggt     720
   atatttgggg ccgggacaac cctgaccgtc ctaggtgcgg ccgc                      764
<210> 25
   <211> 254
   <212> PRT
   <213> Artificial
<220>
   <223> scFv
<400> 25
   Met Ala Ala Val Thr Leu Asp Glu Ser Gly Gly Gly Leu Gln Thr Pro
   1               5                   10                  15
   Gly Gly Gly Phe Ser Leu Val Cys Lys Ala Ser Gly Phe Thr Phe Ser
               20                  25                  30
   Ser Tyr Ala Met Met Trp Val Arg Gln Ala Pro Gly Lys Arg Leu Glu
           35                  40                  45
   Trp Val Ala Gly Ile Ser Gly Ala Gly Ser Tyr Thr Asn Tyr Gly Ala
       50                  55                  60
   Ala Val Lys Gly Arg Ala Thr Ile Ser Arg Asp Asn Gly Gln Ser Thr
   65 70 75 80
   Ala Arg Leu Gln Leu Asn Asn Leu Arg Ala Glu Asp Thr Gly Thr Tyr
   85 90 95
   Phe Cys Ala Arg Ser Ser Gly Tyr Cys Gly Phe Ser Cys Ala Glu Tyr
   100 105 110
   Ile Asp Ala Trp Gly His Gly Thr Glu Val Met Val Ser Ser Gly Ser
   115 120 125
   Ala Ser Ala Pro Lys Leu Glu Glu Gly Glu Phe Ser Glu Ala Arg Val
   130 135 140
   Ala Leu Thr Gln Pro Ser Ser Val Ser Ala Asn Leu Gly Gly Thr Val
   145 150 155 160
   Lys Ile Thr Cys Ser Gly Gly Gly Ser Tyr Tyr Gly Trp Tyr Gln Gln
   165 170 175
   Lys Ala Pro Asn Ser Ala Pro Val Ser Val Ile Tyr Asp Asn Thr Asn
   180 185 190
   Arg Pro Ser Asn Ile Pro Ser Arg Phe Ser Gly Ser Lys Ser Gly Ser
   195 200 205
   Thr Ala Thr Leu Thr Ile Thr Gly Val Gln Ala Asp Asp Glu Ala Val
   210 215 220
   Tyr Phe Cys Gly Ser Tyr Asp Ser Ser Thr Asp Ser Gly Tyr Val Gly
   225 230 235 240
   Ile Phe Gly Ala Gly Thr Thr Leu Thr Val Leu Gly Ala Ala
   245 250
<210> 26
   <211> 758
   <212> DNA
   <213> Artificial
<220>
   <223> scFv encoding DNA of JM_08
<400> 26
   atggccgccg tgacgttgga cgagtccggg ggcggcctcc agaagcccgg aggagggctc 60
   agcctcgtct gcaaggcctc cgggttcccc ttcagcagtt acagcatgca gtgggtgcga 120
   caggcacccg gcaaggggct ggaatgggtc gcaggtattg gcaatgctgg tagttacaca 180
   ggatatgggg cggcggtgaa gggccgtgcc accatctcga gggacaacgg gcagagcaca 240
   gtgaggctgc agctgaacaa cctcagggct gaggacaccg gcacctacta ctgcgccaaa 300
   agtgcttatg ctggtagttg tgctggttgt gttgatagca tcgacgcatg gggccacggg 360
   accgaagtca tggtctcctc cgggagtgca tccgccccaa agcttgaaga aggtgaattt 420
   tcagaagcac gcgtagcgct gactcagccg tcctcggtgt cagcgaaccc gggagaaacc 480
   gtcaagatca cctgctccgg ggatgacagc tggtatggct ggtatcagca gaagtcacct 540
   ggcagtgccc ctgtcactgt gatctattac aacaacaaca gaccctcgga catcccttta 600
   cgattctccg gttccacatc tggctccaca agcacattaa ccatcactgg ggtccaagcc 660
   gacgacgagg ctgtctattt ctgtgggagc tgggacagga gcagtggtgc tggtatattt 720
   ggggccggga caaccctgac cgtcctaggt gcggccgc 758
<210> 27
   <211> 252
   <212> PRT
   <213> Artificial
<220>
   <223> DNA encoding scFv
<400> 27
   Met Ala Ala Val Thr Leu Asp Glu Ser Gly Gly Gly Leu Gln Lys Pro
   1               5                   10                  15
   Gly Gly Gly Leu Ser Leu Val Cys Lys Ala Ser Gly Phe Pro Phe Ser
               20                  25                  30
   Ser Tyr Ser Met Gln Trp Val Arg Gln Ala Pro Gly Lys Gly Leu Glu
           35                  40                  45
   Trp Val Ala Gly Ile Gly Asn Ala Gly Ser Tyr Thr Gly Tyr Gly Ala
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Thr Tyr
                   85                  90                  95
   Tyr Cys Ala Lys Ser Ala Tyr Ala Gly Ser Cys Ala Gly Cys Val Asp
               100                 105                 110
   Ser Ile Asp Ala Trp Gly His Gly Thr Glu Val Met Val Ser Ser Gly
           115                 120                 125
   Ser Ala Ser Ala Pro Lys Leu Glu Glu Gly Glu Phe Ser Glu Ala Arg
   130 135 140
   Val Ala Leu Thr Gln Pro Ser Ser Val Ser Ala Asn Pro Gly Glu Thr
   145 150 155 160
   Val Lys Ile Thr Cys Ser Gly Asp Asp Ser Trp Tyr Gly Trp Tyr Gln
   165 170 175
   Gln Lys Ser Pro Gly Ser Ala Pro Val Thr Val Ile Tyr Tyr Asn Asn
   180 185 190
   Asn Arg Pro Ser Asp Ile Pro Leu Arg Phe Ser Gly Ser Thr Ser Gly
   195 200 205
   Ser Thr Ser Thr Leu Thr Ile Thr Gly Val Gln Ala Asp Asp Glu Ala
   210 215 220
   Val Tyr Phe Cys Gly Ser Trp Asp Arg Ser Ser Gly Ala Gly Ile Phe
   225 230 235 240
   Gly Ala Gly Thr Thr Leu Thr Val Leu Gly Ala Ala
   245 250
<210> 28
   <211> 15
   <212> PRT
   <213> Influenza A virus
<400> 28
   Cys Pro Tyr Gln Gly Thr Pro Ser Phe Phe Arg Asn Val Val Trp
   1 5 10 15
<210> 29
   <211> 15
   <212> PRT
   <213> Influenza A virus
<400> 29
   Gln Gly Thr Pro Ser Phe Phe Arg Asn Val Val Trp Leu Ile Lys
   1 5 10 15
<210> 30
   <211> 15
   <212> PRT
   <213> Influenza A virus
<400> 30
   Pro Ser Phe Phe Arg Asn Val Val Trp Leu Ile Lys Lys Asn Asn
   1 5 10 15
<210> 31
   <211> 15
   <212> PRT
   <213> Influenza A virus
<400> 31
   Phe Arg Asn Val Val Trp Leu Ile Lys Lys Asn Asn Thr Tyr Pro
   1 5 10 15
<210> 32
   <211> 15
   <212> PRT
   <213> Influenza A virus
<400> 32
   Val Val Trp Leu Ile Lys Lys Asn Asn Thr Tyr Pro Thr Ile Lys
   1 5 10 15
<210> 33
   <211> 15
   <212> PRT
   <213> Influenza A virus
<400> 33
   Leu Ile Lys Lys Asn Asn Thr Tyr Pro Thr Ile Lys Arg Ser Tyr
   1 5 10 15
<210> 34
   <211> 15
   <212> PRT
   <213> Influenza A virus
<400> 34
   Lys Asn Asn Thr Tyr Pro Thr Ile Lys Arg Ser Tyr Asn Asn Thr
   1 5 10 15
<210> 35
   <211> 758
   <212> DNA
   <213> Artificial
<220>
   <223> scFv antibody sequence
<220>
   <221> misc_feature
   <222> (675)..(675)
   <223> n is a, c, g, or t
<400> 35
   atggccgccg tgacgttgga cgagtccggg ggcggcctcc agacgcccgg aggagcgctc     60
   agtctcgtct gcaaggcctc cgggttcatc ttcagtgacc gtggcatgca ctgggtgcga    120
   caggcgcccg gcaaggggct ggagtgggtc ggtggtattg gcagcagtgg tagtagcaca    180
   tggtacgggg cggcggtgaa gggccgtgcc acaatctcga gggacaacgg gcagagcaca    240
   gtgaggctgc agctgaacaa cctcagggct gaggacaccg gcacctacta ctgcgccaaa    300
   gatgctagta gtggtggtgg tggtagtcct tatggtgttg ctagaatcga cgcatggggc    360
   cacgggaccg aagtcatggt ctcctccggg agtgcatccg ccccaaagct tgaagaaggt    420
   gaattttcag aagcacgcgt agcgctgact cagccgtcct cggtgtcagc aaacccagga    480
   gaaaccgtca agatcacctg ctccgggagt agtggcagct atggctggta ccagcagaag    540
   tctcctggca gtgcccttgt cactgtgatc tatgaaagca ccaagagacc ctcgaacatc    600
   ccttcacgat tctccggttc caagtccggc tccacagcca cattaaccat cactggggtc    660
   caagccgagg acgangctgt ctattactgt gggagtgcag acagcagtgg tgctatattt    720
   ggggccggga caaccctgac cgtcctaggt gcggccgc                            758
<210> 36
   <211> 252
   <212> PRT
   <213> Artificial
<220>
   <223> scFv antibody sequence
<400> 36
   Met Ala Ala Val Thr Leu Asp Glu Ser Gly Gly Gly Leu Gln Thr Pro
   1               5                   10                  15
   Gly Gly Ala Leu Ser Leu Val Cys Lys Ala Ser Gly Phe Ile Phe Ser
               20                  25                  30
   Asp Arg Gly Met His Trp Val Arg Gln Ala Pro Gly Lys Gly Leu Glu
           35                  40                  45
   Trp Val Gly Gly Ile Gly Ser Ser Gly Ser Ser Thr Trp Tyr Gly Ala
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Thr Tyr
                   85                  90                  95
   Tyr Cys Ala Lys Asp Ala Ser Ser Gly Gly Gly Gly Ser Pro Tyr Gly
               100                 105                 110
   Val Ala Arg Ile Asp Ala Trp Gly His Gly Thr Glu Val Met Val Ser
   115 120 125
   Ser Gly Ser Ala Ser Ala Pro Lys Leu Glu Glu Gly Glu Phe Ser Glu
   130 135 140
   Ala Arg Val Ala Leu Thr Gln Pro Ser Ser Val Ser Ala Asn Pro Gly
   145 150 155 160
   Glu Thr Val Lys Ile Thr Cys Ser Gly Ser Ser Gly Ser Tyr Gly Trp
   165 170 175
   Tyr Gln Gln Lys Ser Pro Gly Ser Ala Leu Val Thr Val Ile Tyr Glu
   180 185 190
   Ser Thr Lys Arg Pro Ser Asn Ile Pro Ser Arg Phe Ser Gly Ser Lys
   195 200 205
   Ser Gly Ser Thr Ala Thr Leu Thr Ile Thr Gly Val Gln Ala Glu Asp
   210 215 220
   Glu Ala Val Tyr Tyr Cys Gly Ser Ala Asp Ser Ser Gly Ala Ile Phe
   225 230 235 240
   Gly Ala Gly Thr Thr Leu Thr Val Leu Ala Ala Ala
   245 250
<210> 37
   <211> 764
   <212> DNA
   <213> Artificial
<220>
   <223> scFV
<400> 37
   atggccgccg tgacgttgga cgagtccggg ggcggcctcc agacgcccgg aggagcgctc     60
   agtctcgtct gcaaggcctc cgggttcatc ttcagtgacc gtggcatgca ctgggtgcga    120
   caggcgcccg gcaaggggct ggagtgggtc ggtggtattg gcagcagtgg tagtagcaca    180
   tggtacgggg cggcggtgaa gggccgtgcc accatctcga gggacaacgg gcagagcaca    240
   gtgaggctgc agctgaacaa cctcagggct gaggacaccg gcacctacta ctgcgccaaa    300
   gatgctagta gtggtggtgg tggtagtcct tatggtgttg ctagaatcga cgcatggggc    360
   cacgggaccg aagtcatggt ctcctccggg agtgcatccg ccccaaagct tgaagaaggt    420
   gaattttcag aagcacgcgt agcgctgact cagccgtcct cggtgtcagc aaacctggga    480
   ggaaccgtcg agatcacctg ctccgggagt agtggcagct atggctggta tcagcagaag    540
   tcacctggca gtgcccctgt ctctgtgatc tatagtaacg acaagagacc ctcggacatc    600
   ccttcacgat tctccggttc caagtccggc tccacagcca cattaatcat cactggggtc    660
   cgagccgagg acgaggctgt ctattactgt gggaattacg acagcagcag tactgctgat    720
   atgtttgggg ccgggacaac cctgaccgtc ctaggtgcgg ccgc                     764
<210> 38
   <211> 254
   <212> PRT
   <213> Artificial
<220>
   <223> scFv antibody sequence
<400> 38
   Met Ala Ala Val Thr Leu Asp Glu Ser Gly Gly Gly Leu Gln Thr Pro
   1               5                   10                  15
   Gly Gly Ala Leu Ser Leu Val Cys Lys Ala Ser Gly Phe Ile Phe Ser
               20                  25                  30
   Asp Arg Gly Met His Trp Val Arg Gln Ala Pro Gly Lys Gly Leu Glu
           35                  40                  45
   Trp Val Gly Gly Ile Gly Ser Ser Gly Ser Ser Thr Trp Tyr Gly Ala
       50                  55                  60
   Ala Val Lys Gly Arg Ala Thr Ile Ser Arg Asp Asn Gly Gln Ser Thr
   65                  70                  75                  80
   Val Arg Leu Gln Leu Asn Asn Leu Arg Ala Glu Asp Thr Gly Thr Tyr
                   85                  90                  95
   Tyr Cys Ala Lys Asp Ala Ser Ser Gly Gly Gly Gly Ser Pro Tyr Gly
               100                 105                 110
   Val Ala Arg Ile Asp Ala Trp Gly His Gly Thr Glu Val Met Val Ser
           115                 120                 125
   Ser Gly Ser Ala Ser Ala Pro Lys Leu Glu Glu Gly Glu Phe Ser Glu
       130                 135                 140
   Ala Arg Val Ala Leu Thr Gln Pro Ser Ser Val Ser Ala Asn Leu Gly
   145                 150                 155                 160
   Gly Thr Val Glu Ile Thr Cys Ser Gly Ser Ser Gly Ser Tyr Gly Trp
                   165                 170                 175
   Tyr Gln Gln Lys Ser Pro Gly Ser Ala Pro Val Ser Val Ile Tyr Ser
   180 185 190
   Asn Asp Lys Arg Pro Ser Asp Ile Pro Ser Arg Phe Ser Gly Ser Lys
   195 200 205
   Ser Gly Ser Thr Ala Thr Leu Ile Ile Thr Gly Val Arg Ala Glu Asp
   210 215 220
   Glu Ala Val Tyr Tyr Cys Gly Asn Tyr Asp Ser Ser Ser Thr Ala Asp
   225 230 235 240
   Met Phe Gly Ala Gly Thr Thr Leu Thr Val Leu Gly Ala Ala
   245 250
<210> 39
<400> 39
   000
<210> 40
   <211> 763
   <212> DNA
   <213> Artificial
<220>
   <223> scFv antibody sequence
<400> 40
   ccatggccgc cgtgacgttg gacgagtccg ggggcggcct ccagacgccc ggaggagcgc 60
   tcagcctcgt ctgcaaggcc tccgggttta ccttcagtga ttacagcgtg cagtgggtgc 120
   gccaggcgcc cggcaaggga ctggagtggg tcgcggggat tcactacact ggcagtagtt 180
   acatgtacta tgcgccggcg gtgaagggcc gtgccaccat ctcgagggac aacgggcaga 240
   gcacagtgag gctgcagctg aacaacctca gggctgagga caccggcacc tacttctgcg 300
   ccaaaagcag tcatggtgat tttggtgatt atgaagcggg tcgtatcgac gcatggggcc 360
   acgggaccga agtcatggtc tcctccggga gtgcatccgc cccaaagctt gaagaaggtg 420
   aattttcaga agcacgcgta gcgctgactc agccgtcctc ggtgtcagca aacccaggag 480
   aaaccatcaa gatcacctgc tcaggcagta gcgatgctta tggctggtac cagcagaagt 540
   ctcctggcag tgcccctgtc actgtgatct ataatggcaa taacagaccc tcggacatcc 600
   cttcacgatt ctccggttcc aaatccggct ccacgggcac attaaccatc actggggtcc 660
   aagccgatga cgaggctgtc tattactgtg ggagcacaga caggaacact aatagtggta 720
   tatttggggc cgggacaacc ctgaccgtcc taggtgcggc cgc 763
<210> 41
   <211> 253
   <212> PRT
   <213> Artificial
<220>
   <223> scFv sequence
<400> 41
   Met Ala Ala Val Thr Leu Asp Glu Ser Gly Gly Gly Leu Gln Thr Pro
   1               5                   10                  15
   Gly Gly Ala Leu Ser Leu Val Cys Lys Ala Ser Gly Phe Thr Phe Ser
               20                  25                  30
   Asp Tyr Ser Val Gln Trp Val Arg Gln Ala Pro Gly Lys Gly Leu Glu
           35                  40                  45
   Trp Val Ala Gly Ile His Tyr Thr Gly Ser Ser Tyr Met Tyr Tyr Ala
       50                  55                  60
   Pro Ala Val Lys Gly Arg Ala Thr Ile Ser Arg Asp Asn Gly Gln Ser
   65                  70                  75                  80
   Thr Val Arg Leu Gln Leu Asn Asn Leu Arg Ala Glu Asp Thr Gly Thr
                   85                  90                  95
   Tyr Phe Cys Ala Lys Ser Ser His Gly Asp Phe Gly Asp Tyr Glu Ala
               100                 105                 110
   Gly Arg Ile Asp Ala Trp Gly His Gly Thr Glu Val Met Val Ser Ser
           115                 120                 125
   Gly Ser Ala Ser Ala Pro Lys Leu Glu Glu Gly Glu Phe Ser Glu Ala
       130                 135                 140
   Arg Val Ala Leu Thr Gln Pro Ser Ser Val Ser Ala Asn Pro Gly Glu
   145                 150                 155                 160
   Thr Ile Lys Ile Thr Cys Ser Gly Ser Ser Asp Ala Tyr Gly Trp Tyr
                   165                 170                 175
   Gln Gln Lys Ser Pro Gly Ser Ala Pro Val Thr Val Ile Tyr Asn Gly
               180                 185                 190
   Asn Asn Arg Pro Ser Asp Ile Pro Ser Arg Phe Ser Gly Ser Lys Ser
           195                 200                 205
   Gly Ser Thr Gly Thr Leu Thr Ile Thr Gly Val Gln Ala Asp Asp Glu
       210                 215                 220
   Ala Val Tyr Tyr Cys Gly Ser Thr Asp Arg Asn Thr Asn Ser Gly Ile
   225                 230                 235                 240
   Phe Gly Ala Gly Thr Thr Leu Thr Val Leu Gly Ala Ala
                   245                 250